# EUROPEAN PATENT SPECIFICATION

(11) **EP 0 573 667 B1**
(45) Date of publication and mention of the grant of the patent: **13.06.2001**
(21) Application number: 93900431.3
(22) Date of filing: 25.12.1992
(51) Int. Cl.: C07D 205/08, C07D 413/06, C07D 417/06, C07D 403/06, C07F 7/18

(54) **PROCESS FOR PRODUCING 4-SUBSTITUTED AZETIDINONE DERIVATIVE**
VERFAHREN ZUR HERSTELLUNG VON 4-SUBSTITUIERTEN AZETIDINON-DERIVATEN
PROCEDE DE PRODUCTION D'UN DERIVE D'AZETIDINONE SUBSTITUE EN POSITION 4

(30) Priority: 26.12.1991 JP 35683091; 28.05.1992 JP 16008092; 23.07.1992 JP 21663192
(43) Date of publication of application: 15.12.1993
(73) Proprietor: NIPPON SODA CO., LTD., Chiyoda-ku, Tokyo 100-8165 (JP)
(72) Inventor: NAKAGAWA, Yuuki Takaoka Plant, Takaoka-shi Toyama 933 (JP); IMAI, Kiyohito Takaoka Plant, Takaoka-shi Toyama 933 (JP); HARA, Tamio Takaoka Plant, Takaoka-shi Toyama 933 (JP)
(74) Representative: von Hellfeld, Axel, Dr. Dipl.-Phys.
(86) International application number: JP9201698
(87) International publication number: WO9313064

(56) References cited:
- JP-A- 55 007 251
- JP-A- 56 142 259
- JP-A- 60 019 764
- JP-A- 62 252 785
- JERRY MARCH: "Advanced Organic Chemistry", WILEY INTERSCIENCE, NEW YORK
- TETRAHEDRON LETTERS, vol. 27, pages 5687 to 5690

## Description

The present invention relates to a process for preparing 4-substituted azetidinone derivatives which are important as an intermediate for the synthesis of carbapenem compounds.

### Background Art

Carboxylic acid derivatives of the general formula [I']: 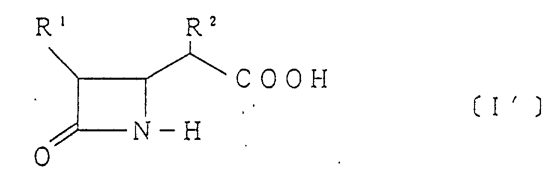 wherein R¹ is alkyl which can be substituted by hydroxy which can be protected or halogen and R² is hydrogen or alkyl, are important as an intermediate for the synthesis of carbapenem compounds and several processes for the preparation thereof have been proposed.

It is known from JP-A-55-7251 to obtain a 4-carbon substituted azetidinone useful as a raw material for antibiotics, by reacting a 2-azetidinone derivative having a lower acyloxy group or halogen at the 4-position with an organomagnesium or organocopper compound.

JP-A-56/142259 discloses β-lactams including 1-(2',4'-dimethoxybenzyl)-3-(1'-acetoxyethyl)-4,4-bisethoxycarbonyl-azetidine-2-one as well as their preparation with a base in a solvent.

JP-A-60-19764 discloses a process for the procuction of azetidinone dervatives wherein a specific azetidinone derivative is reacted with a compound containing a mercapto group.

Among those processes, Jpn. Kokai Tokkyo Koho JP 87252786 discloses the process that a 4-substituted azetidinone of the general formula [II']: 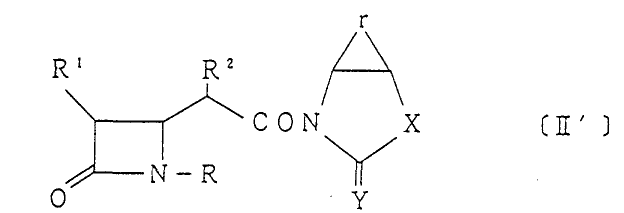 wherein R¹ and R² are as defined above; R is hydrogen or a easily-removable protecting group of nitrogen; r is an unsubstituted or substituted aromatic group formed together with two adjacent carbon atoms; X is oxygen, sulfur, sulfinyl, sulfonyl or Nr¹ group (r¹ is hydrogen, alkyl or phenyl); Y is oxygen, sulfur or Nr² group (r² is hydrogen, alkyl or phenyl), is easily hydrolyzed to a carboxylic acid of the general formula [I'].

Further, Tetrahedron Lett. Vol.27, 5687-5690 (1986) discloses the compound of the general formula [II"]: 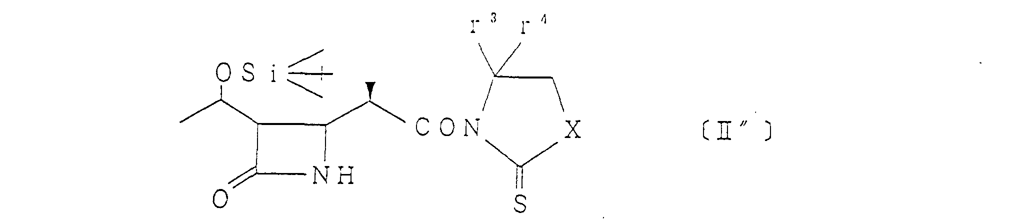 wherein X is as defined above, r³ and r⁴ are independently hydrogen or methyl.

However, these processes for preparing 4-substituted azetidinone derivatives of the general formula [II'] and [II "] do not suit for industrial production because of using expensive boron triflate or tin triflate.

### Disclosure of Invention

This invention relates to a process for preparing 4-substituted azetidinone derivatives of the general formula [II]: 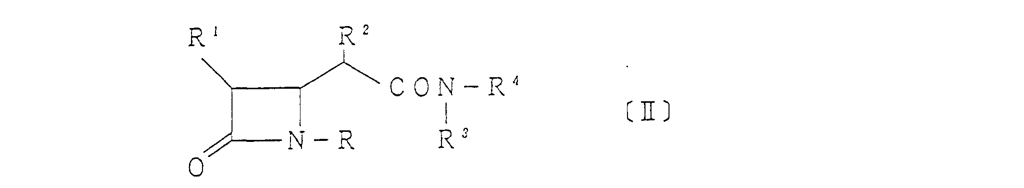 wherein R is hydrogen or an easily removable protecting group for nitrogen atom; R¹ is alkyl which can be substituted by hydroxy which can be protected or halogen; R² is hydrogen or alkyl; R³ is alkyl, trialkylsilyl, phenyl which can be substituted with alkyl, alkoxy, nitro or halogen, cycloalkyl, naphthyl, anthracenyl, fluorenyl, benzothiazolyl, naphthalimidyl; R⁴ is an electron withdrawing group or can form a ring together with R³, which comprises reacting an azetidinone derivative of the general formula [III]: 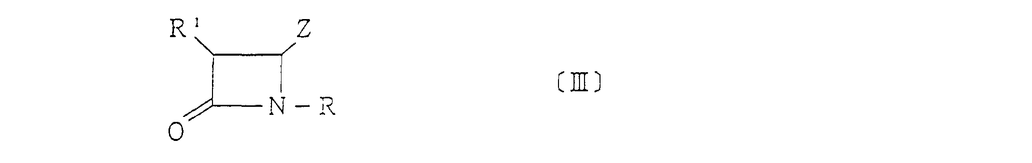 wherein R and R¹ are as defined above, and Z is a leaving group, with an imide compound of the general formula [IV]: 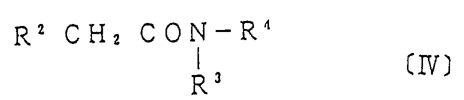 wherein R², R³ and R⁴ are as defined above, characterized in that the process is conducted in the presence of a titanium compound of the general formula [V]:

Ti(Cl) ₙ (OR⁵) ₘ [V]

wherein R⁵ is C₁ to C₃ alkyl; n and m are independently an integer from 0 to 4 provided n plus m makes always 4, and a base

As a protecting group for hydroxy at R¹, for example, organosilyl such as tert-butyldimethylsilyl, tert-butyldiphenylsilyl, triethylsilyl, dimethylcumylsilyl, triisopropylsilyl, dimethylhexylsilyl; oxycarbonyl such as p-nitrobenzyloxycarbonyl, p-methoxybenzyloxycarbonyl, allyloxycarbonyl; acetyl; triphenylmethyl; benzoyl and tetrahydropyranyl are exemplified. As a protecting group for nitrogen, for example, organosilyl as described above, benzyl, p-nitrobenzyl, p-nitrobenzoylmethyl, benzhydryl, p-methoxybenzyl and 2,4-dimethoxybenzyl are exemplified. As a leaving group Z, for example, acyloxy such as normal chain, branching or cyclic alkanoyloxy, monocyclic or bicyclic aroyloxy which can have hetero atoms, alkylsufonyloxy, arylsulfonyloxy, carbamoyloxy, alkoxycarboxy, aralkoxycarboxy and alkoxyalkanoyloxy; acylthio such as alkanoylthio and aroylthio; sulfinyl such as alkylsulfinyl and arylsulfinyl; sulfonyl such as alkylsulfonyl and arylsulfonyl and halogen such as fluorine, chlorine and bromine are exemplified.

As a base, secondary and tertiary amines, anilines and pyridine compounds are used. For example, alkylamines such as dimethylamine, diethylamine, diisopropylamine, dicyclohexylamine, diisopropylethylamine, diisopropylmethylamine, triethylamine; alkylanilines such as N-methylaniline, dimethylaniline; heterocyclic amines such as piporidine, pyrrolidine, 2,2,6,6-tetramethylpiperidine, morpholine piperazine, 1-ethylpiperidine, 1-methylmorpholine, 1-ethylpyrrolidine, 1,4-diazabicyclo[2,2,2]octane and 1,8-diazabicyclo[5,4,0]undec-7-ene; diamine compounds such as N,N,N',N'-tetramethylethylenediamine; alkylpyridines such as α, β or γ-picoline, 2,3-, 2,4-, 2,5-, 2,6-, 3,4- or 3,5-lutidine and 2,4,6-collidine; dialkylaminopyridine such as dimethylaminopyridine and fused heterocyclized pyridines such as Quinoline are exemplified .

As the substituent of the general formula: 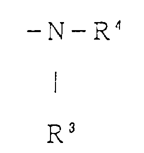 (hereinafter called auxiliary group), the followings can be exemplified. 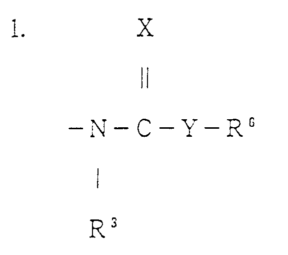
X : O, S, NH, N-alkyl, N-phenyl;
Y : O, S, sulfinyl, sulfonyl, NH, N-alkyl, N-phenyl;
R³ : alkyl(C₃H₇ ⁱ , C₄H₉ ^{t} ), trialkylsilyl 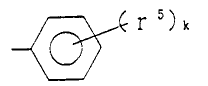 (r⁵: alkyl, halogen, alkoxy, nitro;
k: 0, 1, 2, 3, 4, 5), cycloalkyl, naphthyl, 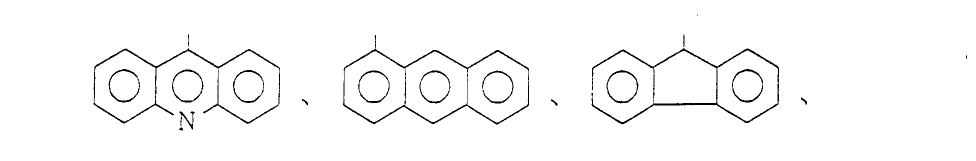 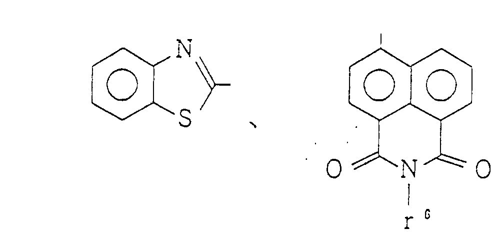 (r⁶ : H, alkyl, phenyl), 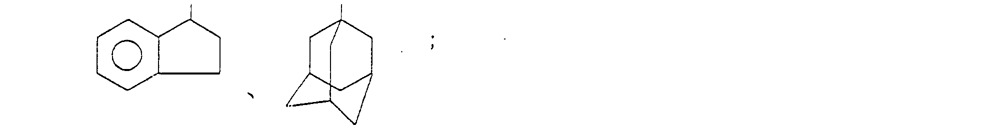
R⁶: alkyl, haloalkyl, 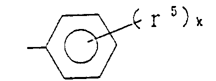 (r⁵, k: as defined above), cycloalkyl, naphthyl:

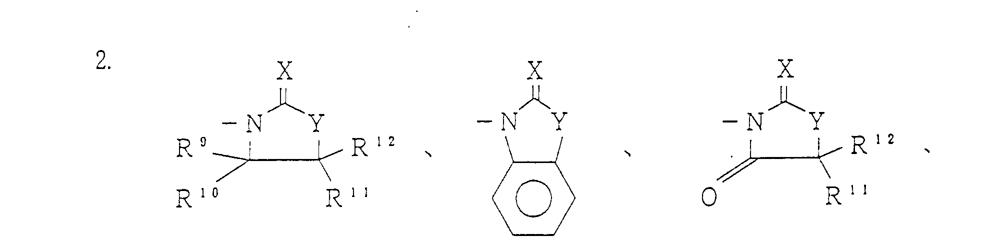

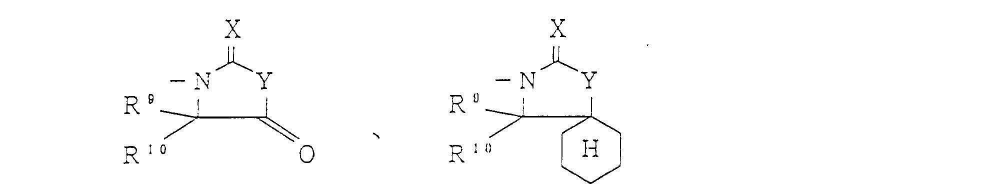

X, Y : As defined above ;
R⁹, R¹⁰, R¹¹, R¹²: H, alkyl, 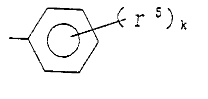 (r⁵, k: as defined above) ,cycloalkyl, naphthyl; 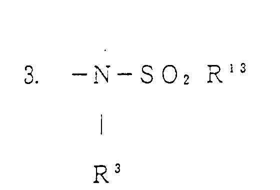
R³ : As defined above; R¹³ : Same as R⁶ defined above; 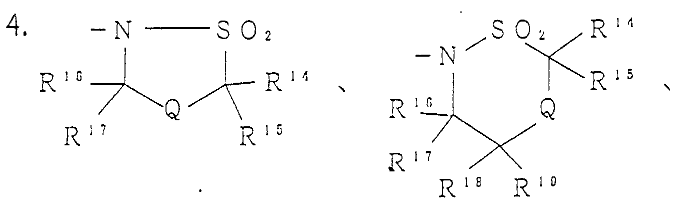 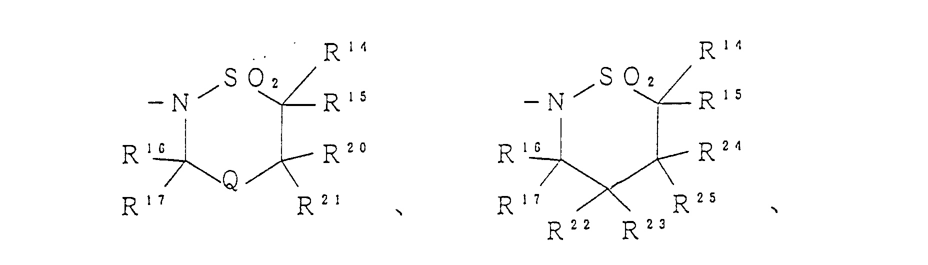 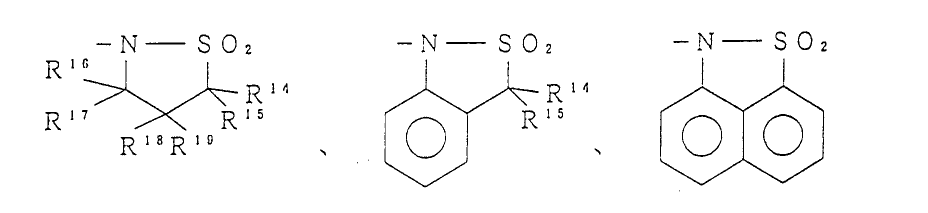 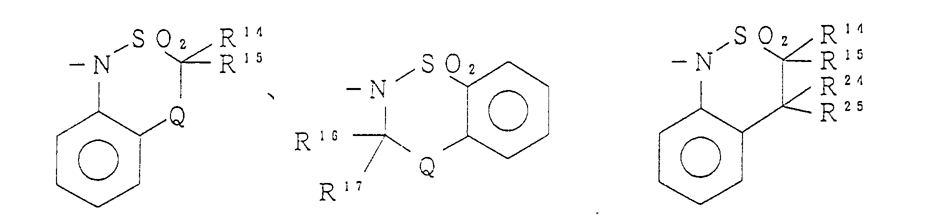 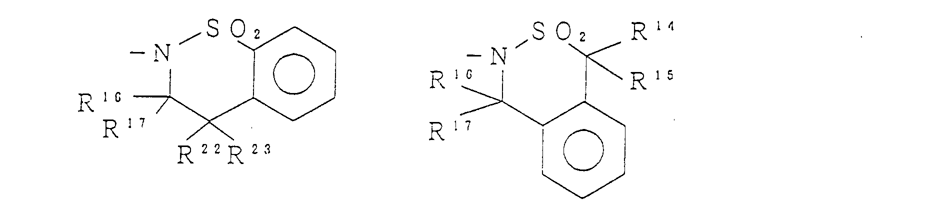 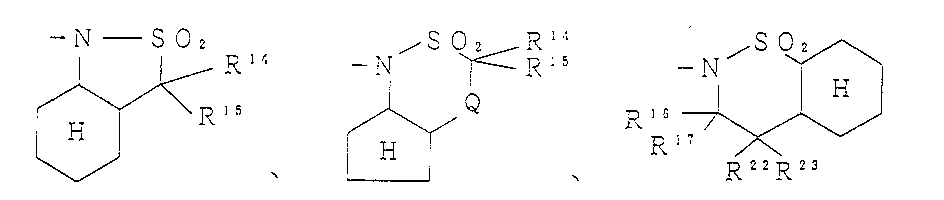
Q : O, S NR²⁶ (R²⁶ : H, alkyl, phenyl);
R¹⁴ ~R²⁵ : H, alkyl, 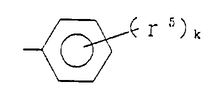 (r⁵, k : as defined above), cycloalkyl, naphthyl; R¹⁴ and R¹⁵, R¹⁶ and R¹⁷, R¹⁸ and R¹⁹, R²⁰ and R²¹, R²² and R²³ and/or R²⁴ and R²⁵ together form oxo or alkylene, respectively; 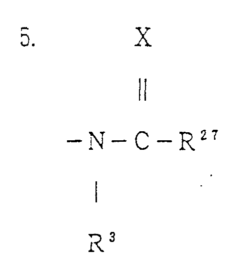
R³, X : As defined above; R²⁷ : Same as R⁶ defined above ; 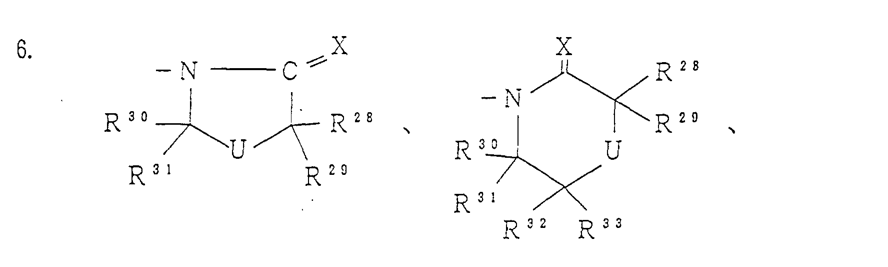 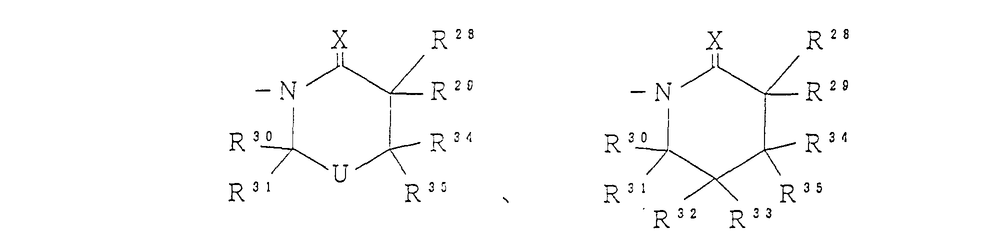 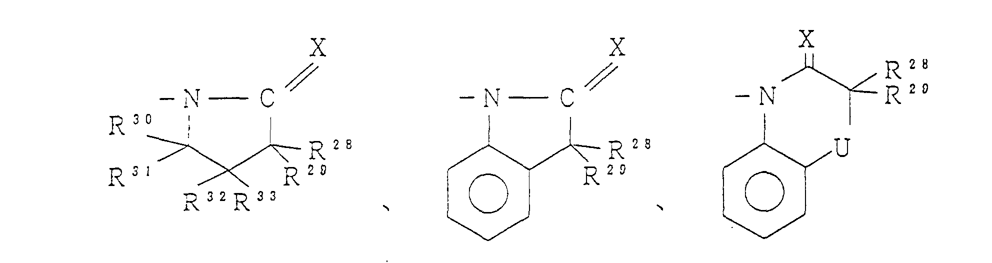 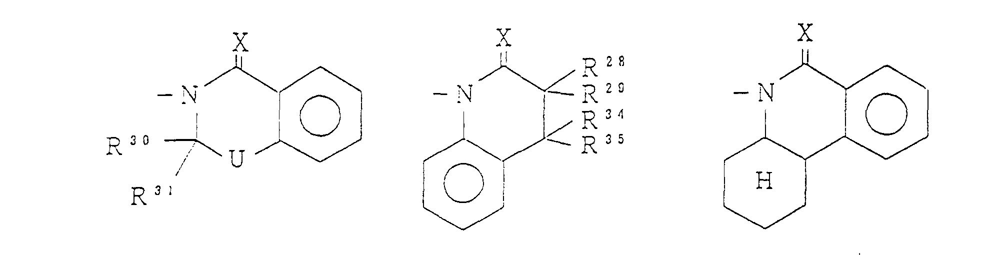 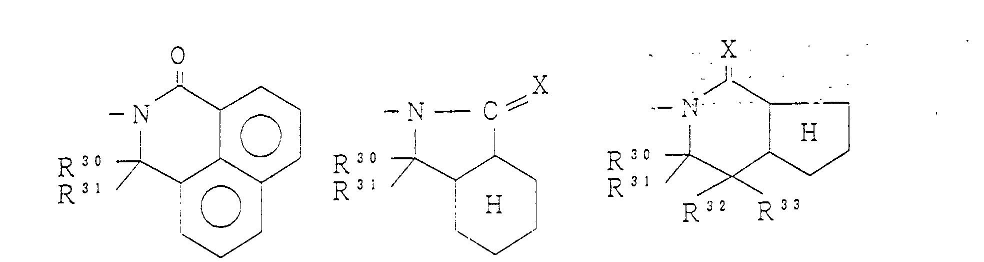
X : As defined above; U: Same as Q defined above;
R²⁸ ∼R³⁵: Same as R¹⁴∼R²⁵ defined above;

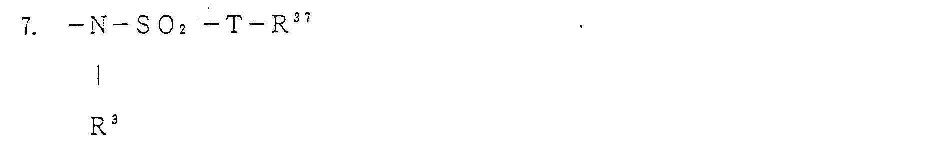

R³ : As defined above;
T : O, S, NR³⁸(R³⁸ : H, alkyl, phenyl);
R³⁷ : Same as R⁶ defined above;

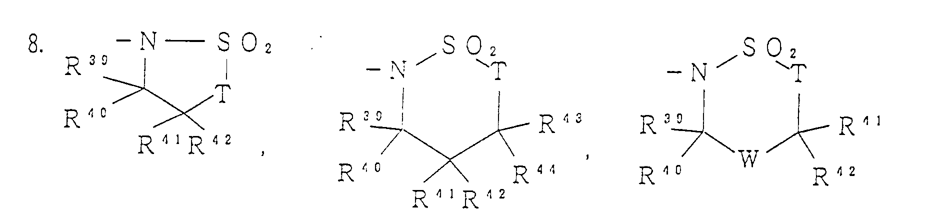

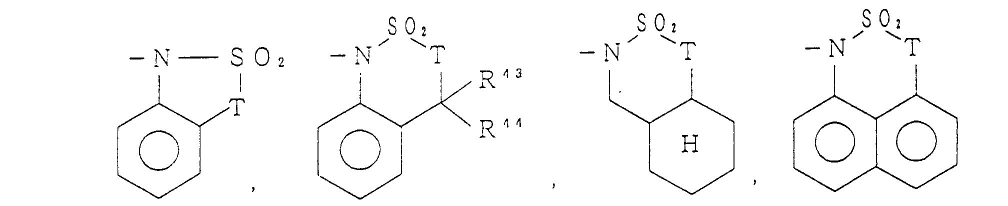

T : As defined above;
W : Same as Q defined above;
R³⁰ ∼R⁴⁴ : Same as R¹⁴ ∼R²⁵ defined above; 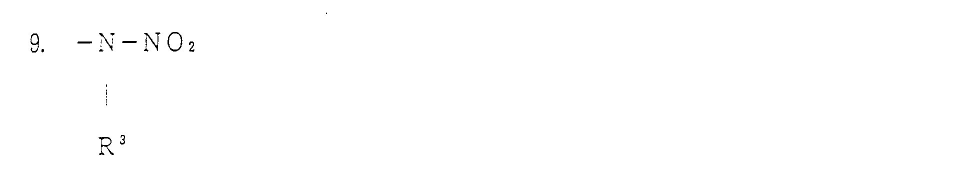
R³ : As defined above;

The reaction is made firstly by reacting an imide compound of the general formula [IV] with a titanium compound of the general formula [V], and a base such as an amine, an aniline or a pyridine compound to produce enolate in organic solvent such as chlorine-containing solvent including methylene chloride, chloroform and the like; aromatic solvent including chlorobenzene, toluene and the like; or polar solvent including acetonitrile and the like, then further reacting the resulting enolate with an azetidinone derivative of the general formula [III]. The reaction temperature for both reaction of enolate formation and of the enolate and an azetidinone derivative is in the range from -50°C to 100 °C,preferably from -20°C to 50°C.

The respective mole ratios of an imide compound of the general formula [IV], a titanium compound [V] and a base to 1 mol of azetidinone of the general formula [III] in the above reaction are 1 to 8 for the all.

Further, when R² is alkyl such as methyl, the ratio of α-type and β-type is influenced by the type of auxiliary group or the mole ratios of the amine and the titanium compound for the imide compound of the general formula [IV]. The formation ratio of the *β* -type can be improved by addition of polar solvent such as DMF, THF and acetonitrile. After the completion of the reaction, the products can be isolated by usual work-up.

It is also possible to convert the products to a carboxylic acid derivative of the general formula [I]: 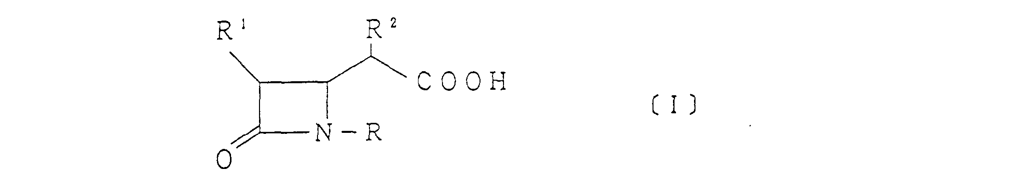 wherein R, R¹ and R² are as defined above, by direct hydrolysis without passing a process of isolation.

### Best Mode for carrying Out the Invention

The present invention is further illustrated in detail according to the following examples.

### EXAMPLE 1

### Preparation of β-methyl derivative (3-[(R)-2-[(3S,4R)-3-[(R)-1-tert-butyldimethylsilyloxyethyl]-2-oxoazetidin-4-yl]propionyl]benzoxazol in-2-one)

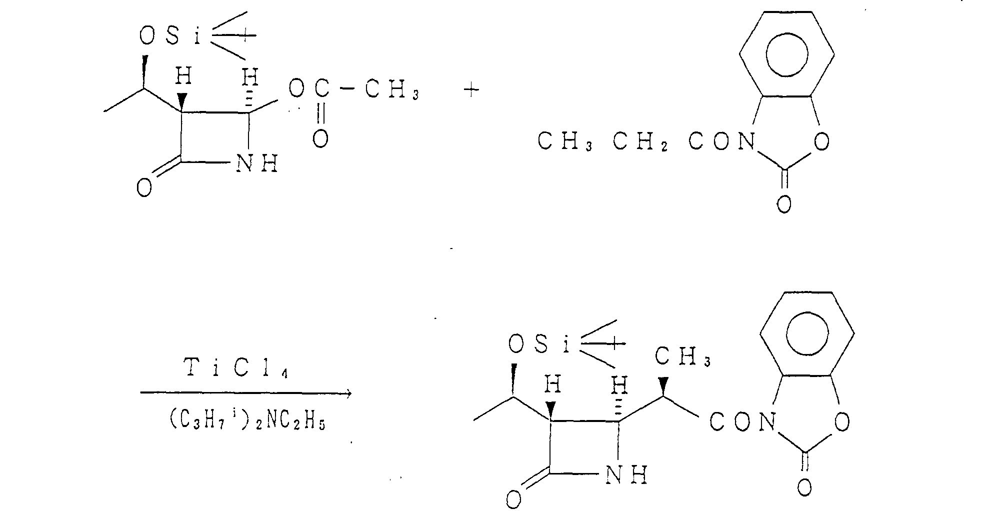

3-Propionylbenzoxazolin-2-one (189mg, 1 mmol) solution in methylene chloride (2 ml) was cooled to -20°C and titanium tetrachloride/methylene chloride solution (1M, 1 ml, 1 mmol) was added to the solution. After aging for 30 minutes at -20°C, N,N-diisopropylethylamine (388 mg, 3 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the solution at the same temperature. After aging the reaction mixture for 2 hours at -20 °C, it was warmed to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 108 mg of the β-methyl derivative (β-methyl derivative: α-methyl derivative= 96:4).

### Example 2

### Preparation of β-methyl derivative (3-[(R)-2-[(3S, 4R)-3-[(R)-1-tert-butyldimethylsilyloxyethyl]-2-oxoazetidin-4-yl]propionyl]-4,4dimethyloxazolidine-2-thione)

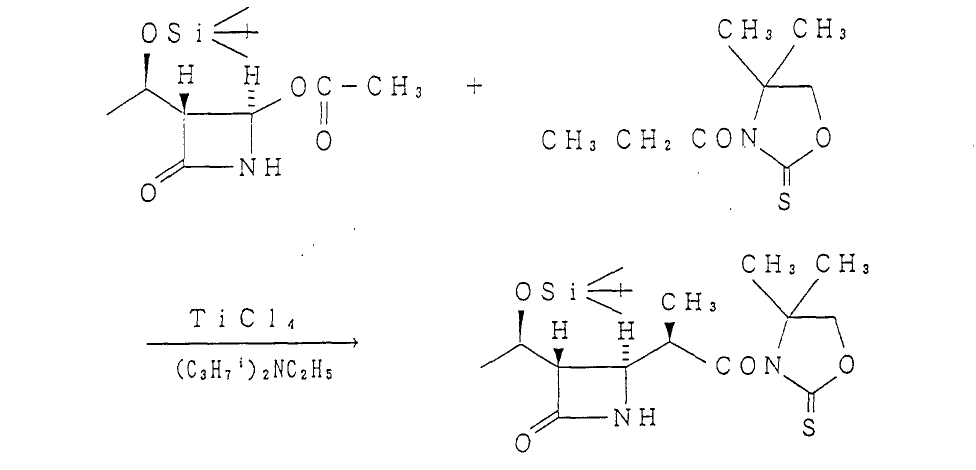

4,4-Dimethyl-3-propionyloxazolidine-2-thione (187 mg, 1 mmol) solution in methylene chloride (2 ml) was cooled to -20°C and titanium tetrachloride/methylene chloride solution (1 M, 1 ml, 1 mmol) was added to the solution. After aging for 30 minutes at -20 °C, N,N-diisopropylethylamine (259 mg, 2 mmol) solution in methylene chloride (1 ml) and (3R, 4R)-4-acetoxy-3-[(R) -1-tert-butyldimethylsilyloxyethyl]azetidin-2-one (287.5 mg, 1 mmol) solution in methylene chloride (1 ml) were added to the solution at the same temperature. After aging the reaction mixture for 1 hour at -20°C, it was warmed to 20 °C and further stirred for 8 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 196 mg of the β-methyl derivative (β-methyl derivative: α-methyl derivative= 95:5).

### Example 3

### Preparation of β-methyl derivative (3-[(R)-2-[(3S, 4R)-3-[(R)-1-tert-butyldimethylsilyloxyethyl]-2-oxoazetidin-4-yl]propionyl]-4,4-dimethyloxazolidine-2-thione)

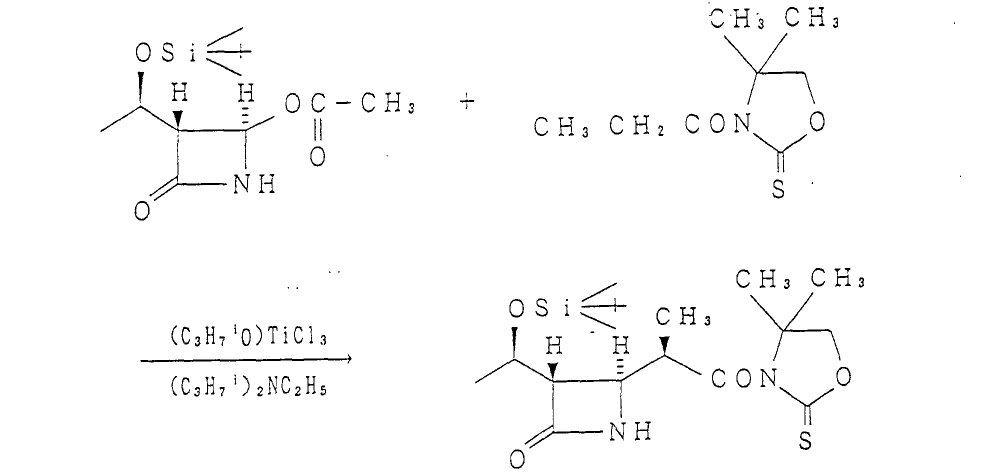

Tetraisopropoxytitanium (71 mg, 0.25 mmol) was added to titanium tetrachloride/methylene chloride solution (1 M, 0.75 ml, 0.75 mmol) at room temperature, then trichloroisopropoxytitanium solution was prepared by aging the above mixture for 2 hours at the same temperature. The reaction mixture was cooled to 0 °C, then 4,4-dimethyl-3-propionyloxazolidine-2-thione (187 mg, 1 mmol) solution in methylene chloride (2 ml) was added to the solution at the same temperature. After aging for 30 minutes at 0°C, N,N-diisopropylethylamine (259 mg, 2 mmol) solution in methylene chloride (1 ml) was added to the solution at the same temperature. Further aging for 30 min. at 0 °C, (3R, 4R)-4-acetoxy-3-[(R)-1-tert-butyldimethylsilyloxyethyl]azetidin-2-one (287.5 mg, 1 mmol) solution in methylene chloride (1 ml) was added to the solution at the same temperature. After aging the reaction mixture for 8 hours at 0 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 118 mg of the β-methyl derivative (β-methyl derivative: α-methyl derivative= 81:19).

### Example 4

### Preparation of β-methyl derivative (3-[(R)-2-[(3S, 4R)-3-[(R)-1-tertbutyldimethylsilyloxyethyl]-2-oxoazetidin-4-yl]propionyl]-4,4-dimethyloxazolidine-2-thione)

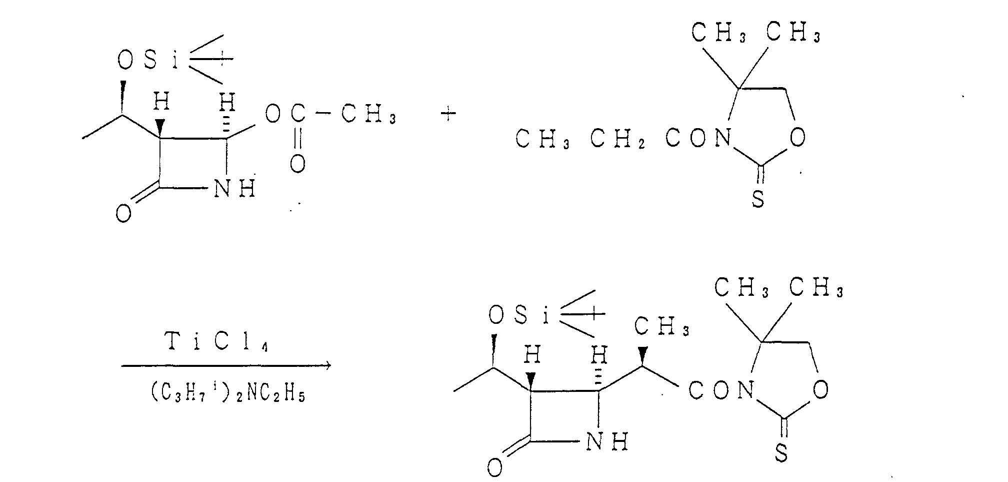

4,4-Dimethyl-3-propionyloxazolidine-2-thione (375 mg, 2 mmol) solution in methylene chloride (2 ml) was cooled to -20°C, then titanium tetrachloride/methylene chloride solution (1 M, 2 ml, 2 mmol) was added to the solution. After aging for 30 minutes at -20 °C, N,N-diisopropylethylamine (259 mg, 2 mmol) solution in methylene chloride (1 ml) and (3R, 4R)-4-acetoxy-3-[(R) -1-tert-butyldimethylsilyloxyethyl]azetidin-2-one (287.5 mg, 1 mmol) solution in methylene chloride (1 ml) were added to the solution at the same temperature. After aging the reaction mixture for 1 hour at -20 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 334 mg of the β-methyl derivative (β-methyl derivative: α-methyl derivative= 97:3).

### Example 5

### Preparation of β-methyl derivative (3-[(R)-2-[(3S, 4R)-3-[(R)-1-tert-butyldimethylsilyoxyethyl]-2-oxoazetidin-4-yl]propionyl]-4,4-dimethylthiazolidine-2-thione)

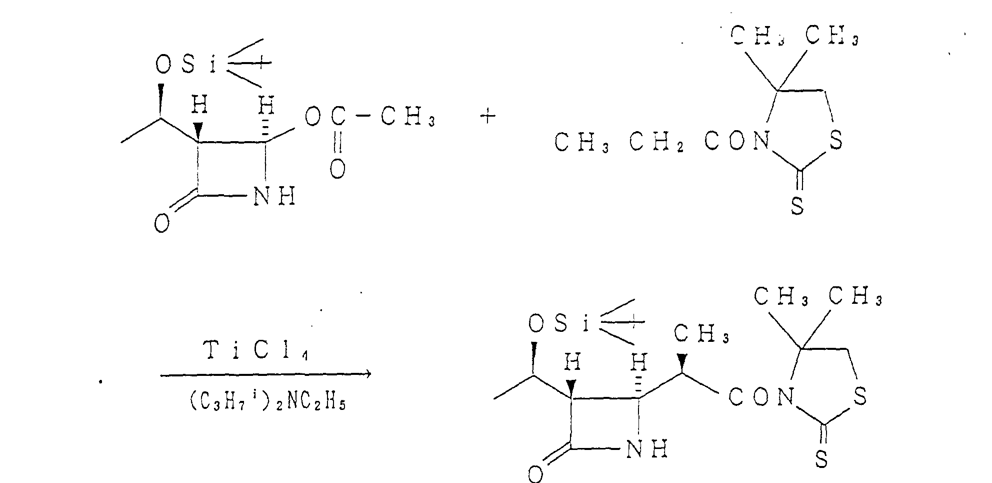

4,4-Dimethyl-3-propionylthiazolidine-2-thione (407 mg, 2 mmol) solution in methylene chloride (2 ml) was cooled to -20 °C, then titanium tetrachloride/methylene chloride solution (1M, 2 ml, 2 mmol) was added to the solution. After aging for 30 minutes at -20°C, N,N-diisopropylethylamine (259 mg, 2 mmol) solution in methylene chloride (1 ml) and (3R, 4R)-4-acetoxy-3-[(R) -1-tert-butyldimethylsilyloxyethyl]azetidin-2-one (287.5 mg, 1 mmol) solution in methylene chloride (1 ml) were added to the solution at the same temperature. After aging the reaction mixture for 1 hour at -20 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 150 mg of the β-methyl derivative (β-methyl derivative: α-methyl derivative= 61:39).

### Example 6

### Preparation of β-methyl derivative (3-[(R)-2-[(3S, 4R)-3-[(R)-1-tert-butyldimethylsilyloxyethyl]-2-oxoazetidin-4-yl]propionyl]-4,4-dimethylthiazolidin-2-one)

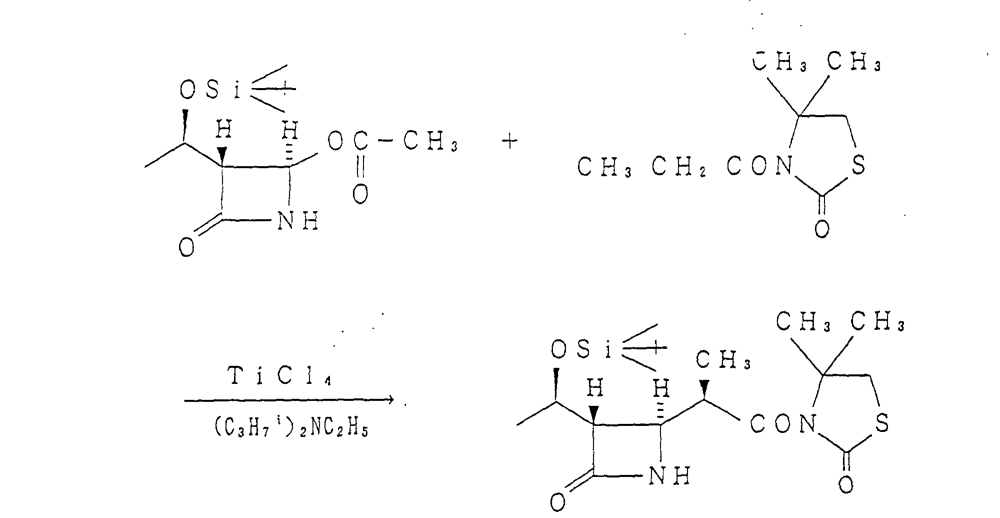

4,4-Dimethyl-3-propionylthiazolidin-2-one (375 mg, 2 mmol) solution in methylene chloride (2 ml) was cooled to -20 °C, then titanium tetrachloride/methylene chloride solution (1 M, 2 ml, 2 mmol) was added to the solution. After aging for 30 minutes at -20 °C, N,N-diisopropylethylamine (259 mg, 2 mmol) solution in methylene chloride (1 ml) and (3R, 4R)-4-acetoxy-3-[(R) -1-tert-butyldimethylsilyloxyethyl]azetidin-2-one (287.5 mg, 1 mmol) solution in methylene chloride (1 ml) were added to the solution at the same temperature. After aging the reaction mixture for 1 hour at -20 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 247 mg of the β-methyl derivative (β-methyl derivative: α-methyl derivative= 81:19).

### Example 7

### Preparation of (3-[(3S, 4R)-3-[(R)-1-tert-butyldimethylsilyloxyethyl]-2-oxoazetidin-4-yl]acetyl-4,4-dimethyloxazolidine-2-thione)

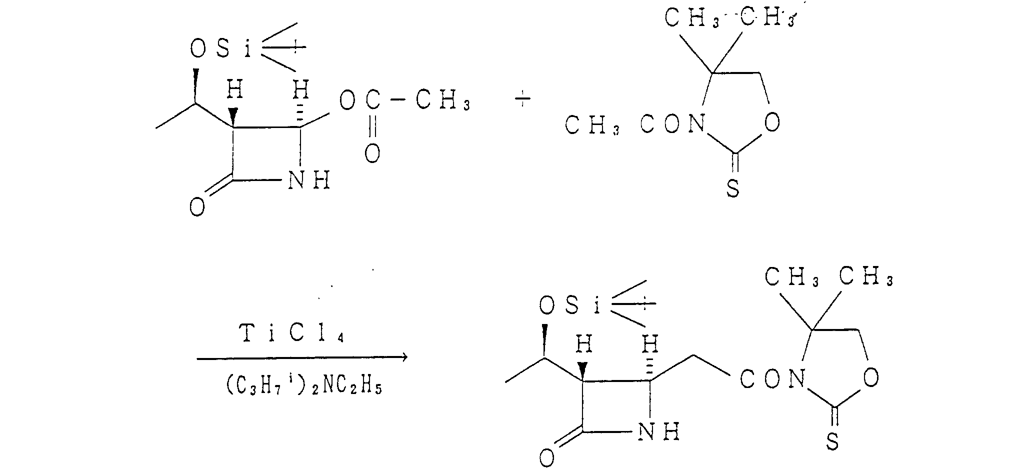

3-Acetyl-4,4-dimethyloxazolidine-2-thione (375 mg, 2 mmol) solution in methylene chloride (2 ml) was cooled to 0 °C, then titanium tetrachloride/methylene chloride solution (1 M, 2 ml, 2 mmol) was added to the solution. After aging for 15 minutes at 0 °C, N,N-diisopropylethylamine (259 mg, 2 mmol) solution in methylene chloride (1 ml) and (3R, 4R)-4-acetoxy-3-[(R) -1-tert-butyldimethylsilyloxyethyl]azetidin-2-one (287.5 mg, 1 mmol) solution in methylene chloride (1 ml) were added to the solution at the same temperature. After aging the reaction mixture for 1 hour at 0 °C, it was warmed to 20 °C and further stirred for 3 hours. The resulting mixture solution was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 280 mg of the product.

### Example 8

### Preparation of β-methyl derivative (3-[(R)-2-[(3S, 4R)-3-[(R)-1-tert-butyldimethylsilyloxyethyl]-2-oxoazetidin-4-yl]propionyl]-4,4-dimethyloxazolidine-2-thione)

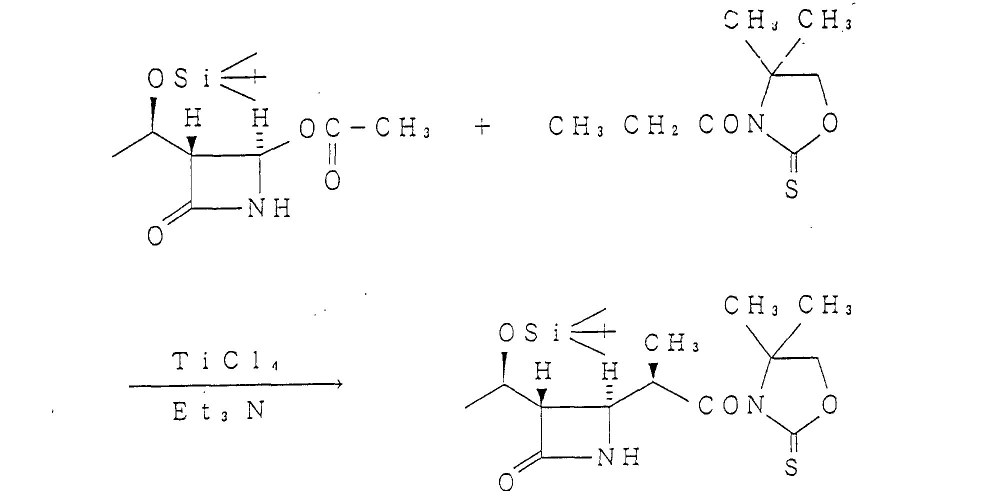

After addition of titanium tetrachloride (256 g, 1.35 mol) to 4,4-dimethyl-3-propionyloxazolidine-2-thione (243 g, 1.3 mol) solution in methylene chloride (4 1) in the temperature range from 20 to 35°C, triethylamine (126 g, 1.25 mol) and (3R, 4R)-4-acetoxy-3-[(R)-1-tert-butyldimethylsilyloxyethyl]azetidin-2-one (287.5 g, 1 mol) were added to the mixture at the same temperature. After aging the reaction mixture for 0.5 hour under reflux, it was cooled to 0 °C and added to water at the same temperature under stirring, then stirred for 0.5 hour under the same temperature. The organic layer was separated and analyzed by using HPLC. The result showed that the organic layer contained 323 g of the β-methyl derivative (β-methyl derivative: α-methyl derivative = 97:3). The organic layer was washed with water (1 1) and added with Isoper G® (Exxon Chemical)(4 1), then the organic layer was concentrated so as to be 3.6 Kg in total weight under reduced pressure. The concentrate was cooled to 5°C under stirring and further stirred for 0.5 hour at the same temperature. The crystals deposited were taken out by filtration and dried, affording 307 g of the β-methyl derivative (β-methyl derivative : α-methyl derivative = 98.5: 1.5).

### Example 9

### Preparation of β-methyl derivative (3-[(R)-2-[(3S, 4R)-3-[(R)-1-tert-butyldimethylsilyloxyethyl]-2-oxoazetidin-4-yl]propionyl]oxazolidine-2-thione)

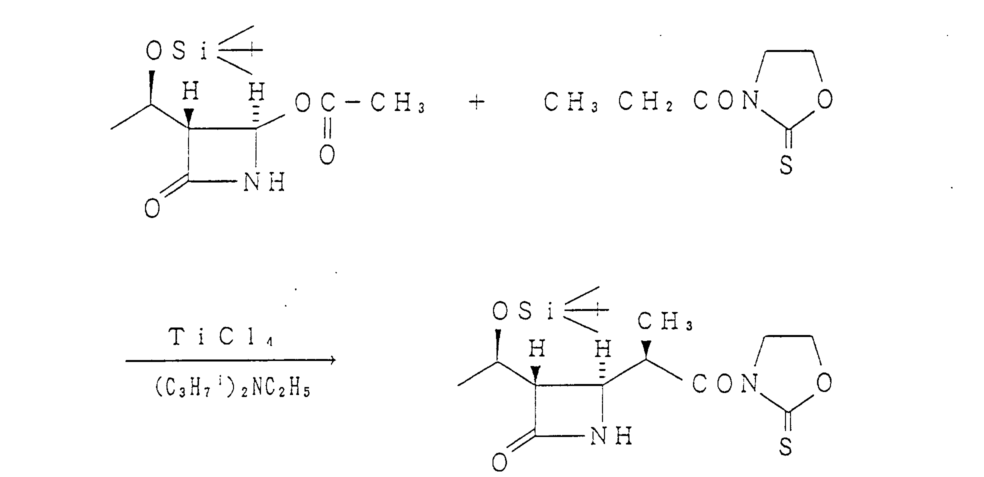

3-Propionyloxazolidine-2-thione (320 mg, 2 mmol) solution in methylene chloride (2 ml) was cooled to 0 °C, then titanium tetrachloride/methylene chloride solution (1 M, 2 ml, 2 mmol) was added to the solution. After aging for 15 minutes at 0 °C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the solution at the same temperature. After aging the reaction mixture for 1 hour at 0°C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 58 mg of the β-methyl derivative (β-methyl derivative : α-methyl derivative = 65:35).

### Example 10

### Preparation of α-methyl derivative (3-[(S)-2-[(3S, 4R)-3-[(R)-1-tert-butyldimethylsilyloxyethyl]-2-oxoazetidin-4-yl]propionyl]-4,4-dimethyloxazolidin-2-one)

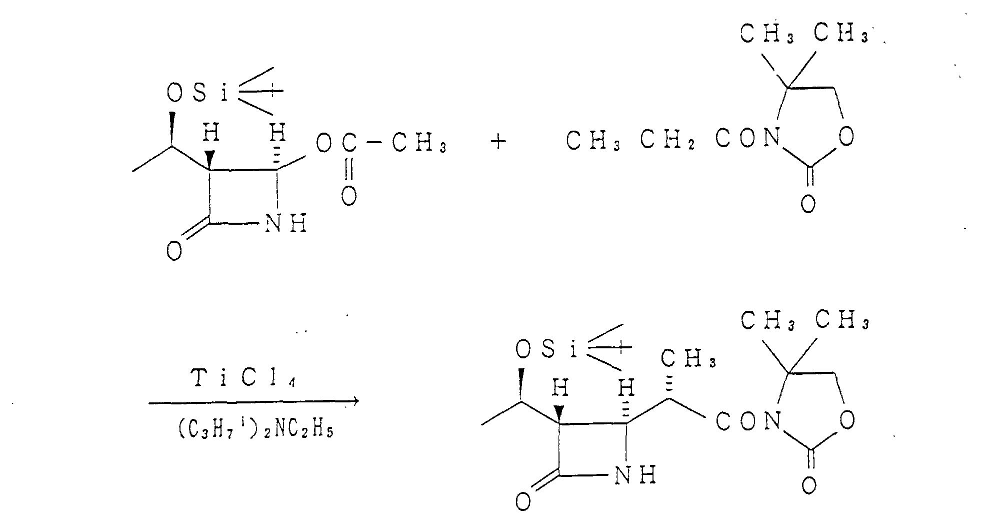

4,4-Dimethyl-3-propionyloxazolidin-2-one (342 mg, 2 mmol) solution in methylene chloride (2 ml) was cooled to -20°C, then titanium tetrachloride/methylene chloride solution (1 M, 2 ml, 2 mmol) was added to the solution. After aging for 30 min. at -20°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the solution at the same temperature. After aging the reaction mixture for 1 hour at -20 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 180 mg of the α-methyl derivative (α-methyl derivative: β-methyl derivative= 68:32).

### Example 11

### Preparation of α-methyl derivative (3-[(S)-2-[(3S, 4R)-3-[(R)-1-tert-butyldimethylsilyloxyethyl]-2-oxoazetidin-4-yl]propionyl]benzoxazolin-2-one)

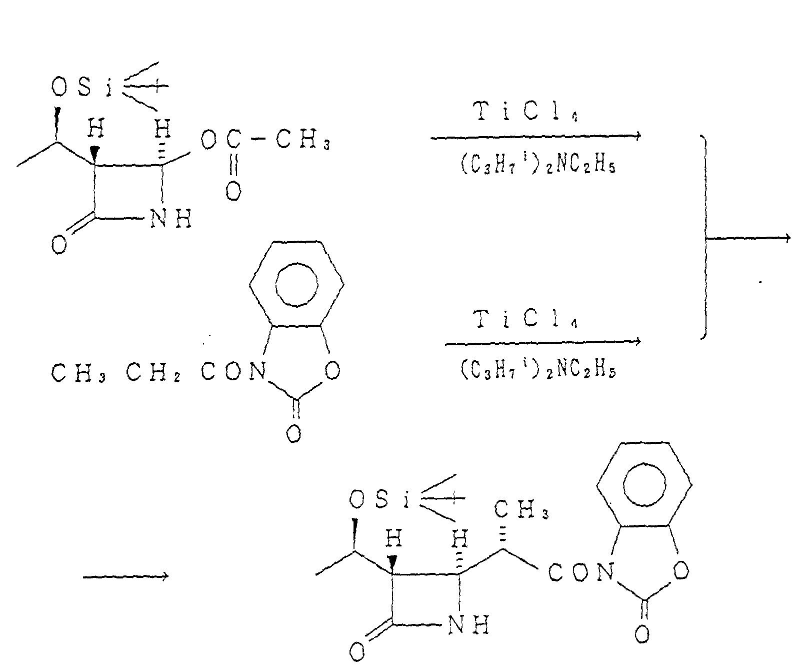

The solution of 3-propionylbenzoxazolin-2-one (3.16 g, 16.5 mmol) and N,N-diisopropylethylamine (4.27 g, 33 mmol) in methylene chloride (20 ml) was cooled to -5 °C, then titanium tetrachloride (3.13 g, 16.5 mmol) solution in methylene chloride (5 ml) was added to the mixture and the reaction mixture was stirred for 15 min. at the same temperature to prepare titanium enolate solution. Besides, titanium tetrachloride (3.13 g, 16.5 mmol) solution in methylene chloride (15 ml) was cooled to -5°C, then the methylene chloride solutions of both N,N-diisopropylethylamine (0.1 g, 0.8 mmol) and (3R, 4R)-4-acetoxy-3-[(R)-1-tert-butyldimethylsilyloxyethyl]azetidin-2-one (4.31 g, 15 mmol) were added thereto at the same temperature. Then the titanium enolate solution previously prepared was added to the resulting mixture and the readtion mixture was stirred for 5 hours at the same temperature, then 10 % sodium hydrogen carbonate aq. solution (100 ml) was added under stirring. The insoluble matter was removed by filtration, the organic layer separated from the filtrate was analyzed by using HPLC. The result showed that the organic layer contained 3.45 g of the α-methyl derivative (α-methyl derivative: β-methyl derivative= 92:8).

### Example 12

### Preparation of β-methyl derivative (3-[(R)-2-[(3S, 4R)-3-[(R)-1-tert-butyldimethylsilyloxyethyl]-2-oxoazetidin-4-yl]propionyl]-4,4-diethyloxazolidine-2-thione)

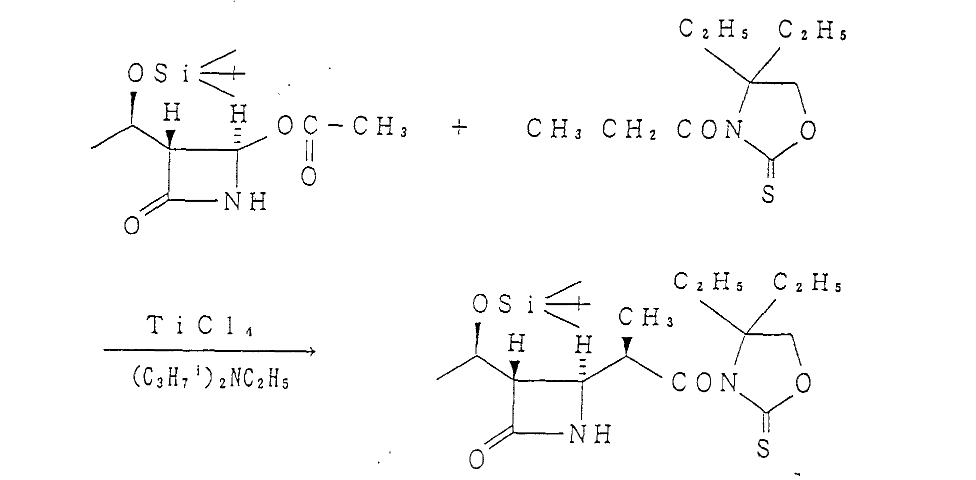

4,4-Diethyl-3-propionyloxazolidine-2-thione (431 mg, 2 mmol) solution in methylene chloride (2 ml) was cooled to 0 °C, then titanium tetrachloride/methylene chloride solution (1 M, 2 ml, 2 mmol) was added to the solution. After aging for 15 min. at 0°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the mixture at the same temperature. After aging the reaction mixture for 1 hour at 0 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 332 mg of the β-methyl derivative (β-methyl derivative: α-methyl derivative= 96:4).

### Example 13

### Preparation of β-methyl derivative (3-[(R)-2-[(3S, 4R)-3-[(R)-1-tertbutyldimethylsilyloxyethyl]-2-oxoazetidin-4-yl]propionyl]-(S)-4-isopropyloxazolidine-2-thione)

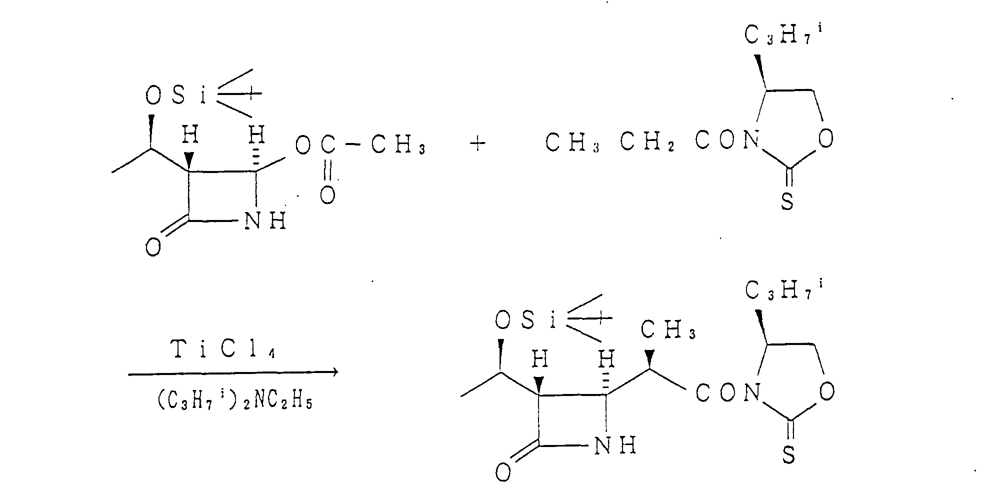

(S)-4-Isopropyl-3-propionyloxazolidine-2-thione (402 mg, 2 mmol) solution in methylene chloride (2 ml) was cooled to 0 °C, then titanium tetrachloride/methylene chloride solution (1 M 2 ml, 2 mmol) was added to the solution. After aging for 15 min. at 0°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the mixture at the same temperature. After aging the reaction mixture for 1 hour at 0 °C, it was warmed to 20 °C and further stirred for 3 hours. The reaction mixture was then cooled to 0°C and 10 % sodium hydrogen carbonate aq. solution (10 ml) was added under stirring. The insoluble matter was removed by filtration, the organic layer separated from the filtrate was analyzed by using HPLC. The result showed that the organic layer contained 203 mg of the β-methyl derivative (β-methyl derivative: α-methyl derivative= 100:0).

### Example 14

### Preparation of β-methyl derivative (3-[(R)-2-[(3S, 4R)-3-[(R)-1-tert-butyldimethylsilyloxyethyl]-2-oxoazetidin-4-yl]propionyl]-4,4-dibutyl-5,5-pentamethyleneoxazolidine-2-thione)

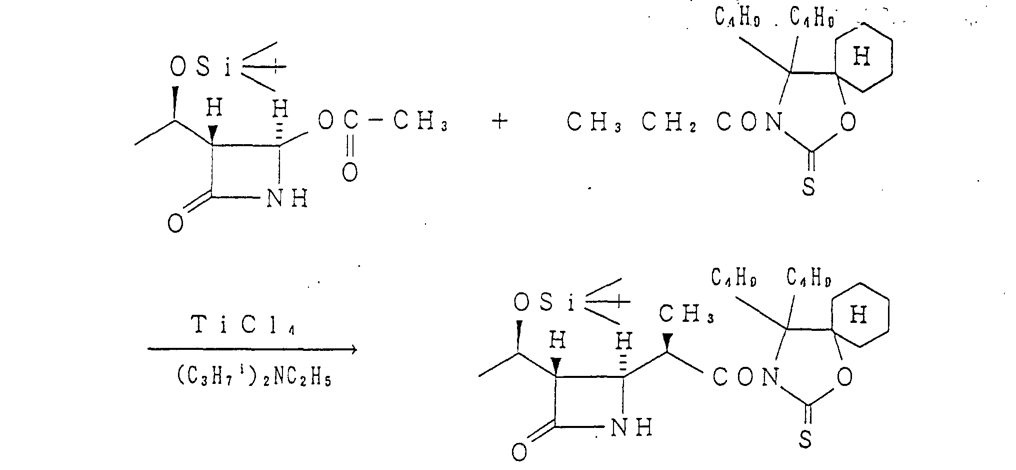

4,4-Dibutyl-5,5-pentamethylene-3-propionyloxazolidine-2-thione (678 mg, 2 mmol) solution in methylene chloride (2 ml) was cooled to -10°C, then titanium tetrachloride/methylene chloride solution (1 M, 2 ml, 2 mmol) was added to the solution. After aging for 15 min. at -10°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the mixture at the same temperature. After aging the reaction mixture for 1 hour at 0 °C, it was warmed to the reflux temperature and further stirred for 3 hours. The reaction mixture was then cooled to 0 °C and 10 % sodium hydrogen carbonate aq. solution (10 ml) was added under stirring. The insoluble matter was removed by filtration, the organic layer separated from the filtrate was analyzed by using HPLC. The result showed that the organic layer contained 334 mg of the β-methyl derivative (β-methyl derivative: α-methyl derivative= 100:0).

### Example 15

### Preparation of α-methyl derivative (1-[(S)-2-[(3S, 4R)-3-[(R)-1-tert-butyldimethylsilyloxyethyl]-2-oxoazetidin-4-yl]propionyl]-3,5,5-trimethylimidazolidine-2,4-dione)

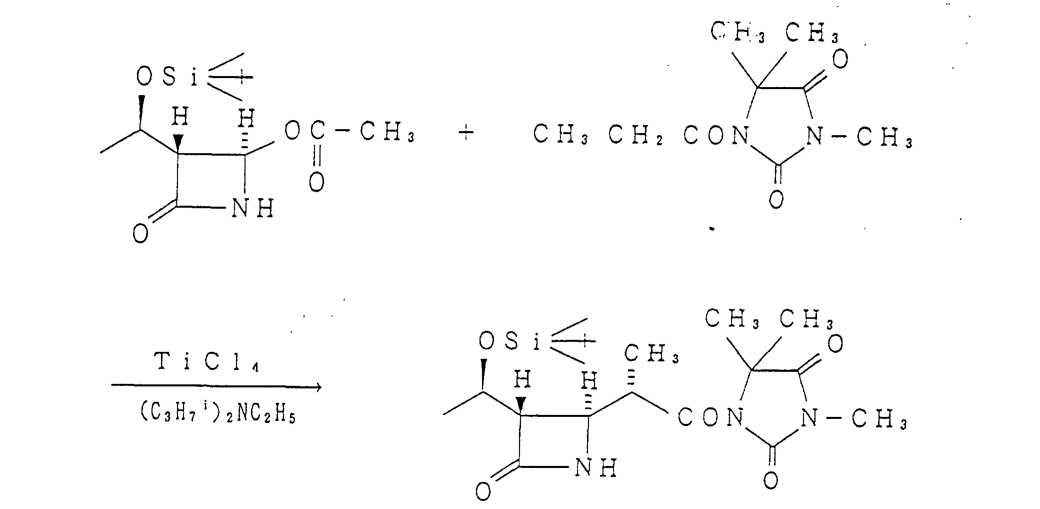

1-Propionyl-3,5,5-trimethylimidazolidine-2,4-dione (397 mg, 2 mmol) solution in methylene chloride (2 ml) was cooled to 0°C, then titanium tetrachloride/methylene chloride solution (1 M, 2 ml, 2 mmol) was added to the solution. After aging for 15 min. at 0°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the mixture at the same temperature. After aging the reaction mixture for 1 hour at 0 °C, it was warmed to 20 °C and further stirred for 3 hours. The reaction mixture was then cooled to 0°C and 10 % sodium hydrogen carbonate aq. solution (10 ml) was added under stirring.

The insoluble matter was removed by filtration, the organic layer separated from the filtrate was analyzed by using HPLC. The result showed that the organic layer contained 190 mg of the α-methyl derivative (α-methyl derivative: *β*-methyl derivative= 92:8).

¹H NMR (270MHz, CDCl₃) *δ* of α-methyl derivative : 0.08(6H,s), 0.89(9H,s), 1.23(3H, d), 1.26(3H,d), 1.64(3H,s), 1.67(3H,s), 2.81(1H,dd), 3.09(3H,s), 3.6 ∼ 4.3(3H,m), 5.99(1H, s)

### Example 16

### Preparation of β-methyl derivative (methyl N-[(R)-2-[(3S, 4R)-3-[(R)-1-tert-butyldimethylsilyloxyethyl]-2-oxoazetidin-4-yl]propionyl]-N-methylthiocarbamate)

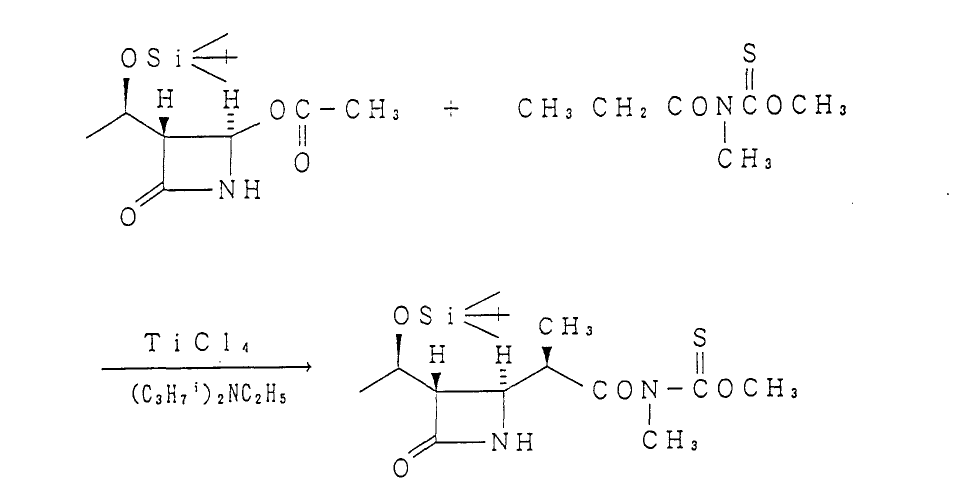

Methyl N-methyl-N-propionylthiocarbamate (1.61 g, 10 mmol) solution in methylene chloride (30 ml) was cooled to 0°C, then titanium tetrachloride (1.9 g, 10 mmol) solution in methylene chloride (5 ml) was added to the solution. After aging for 15 min. at 0 °C, N,N-diisopropylethylamine (1.3 g, 10 mmol) solution in methylene chloride (5 ml) and (3R, 4R)-4-acetoxy-3-[(R)-1-tert-butyldimethylsilyloxyethyl]azetidin-2-one (1.44 g, 5 mmol) solution in methylene chloride (10 ml) were added to the mixture at the same temperature. After aging the reaction mixture for 1 hour at 0°C, it was warmed to 20 °C and further stirred for 3 hours. The reaction mixture was then cooled to 0°C and 10 % sodium hydrogen carbonate aq. solution (50 ml) was added thereto under stirring. The insoluble matter was removed by filtration, the organic layer separated from the filtrate was analyzed by using HPLC. The result showed that the organic layer contained 1.35 g of the β-methyl derivative (β-methyl derivative: α-methyl derivative= 87:13). The organic layer was concentrated and purified by using silica gel column chromatography, affording the purified β-methyl derivative.

The melting point of the β-methyl derivative : 156∼157 °C

### Example 17

### Preparation of β-methyl derivative (methyl N-tert-butyl-N-[(R)-2-[(3S, 4R)-3-[(R)-1-tert-butyldimethylsilyloxyethyl]-2-oxoazetidin-4-yl] propionyl]thiocarbamate)

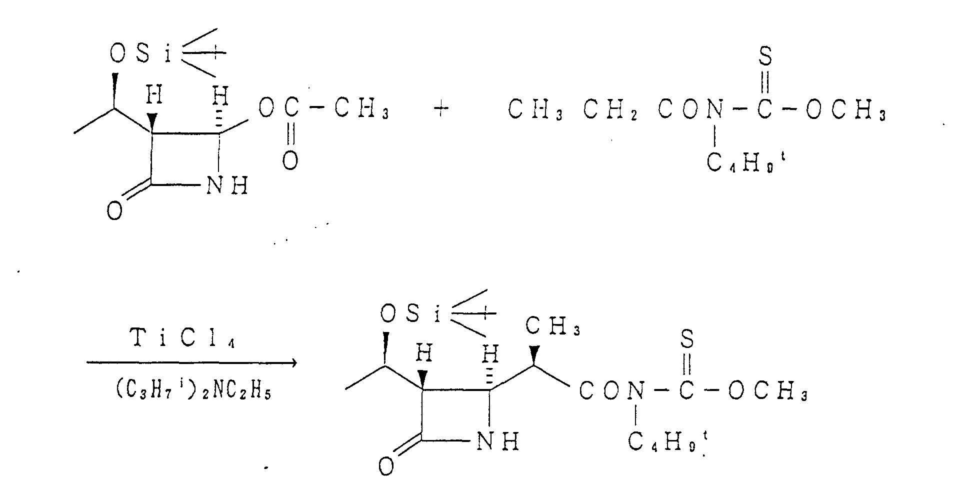

Methyl N-tert-butyl-N-propionylthiocarbamate (1.0 g, 4.9 mmol) solution in methylene chloride (30 ml) was cooled to 0 °C, then titanium tetrachloride (0.93 g, 4.9 mmol) solution in methylene chloride (5 ml) was added to the solution. After aging for 15 min. at 0 °C, N,N-diisopropylethylamine (0.64 g, 4. 9 mmol) solution in methylene chloride (5 ml) and (3R, 4R)-4-acetoxy-3-[(R)-1-tert-butyldimethylsilyloxyethyl]azetidin-2-one (0.71 g, 2.5 mmol) solution in methylene chloride (10 ml) were added to the mixtrue at the same temperature. After aging the reaction mixture for 1 hour at 0°C, it was warmed to 20 °C and further stirred for 3 hours. The reaction mixture was then cooled to 0°C and 10 % sodium hydrogen carbonate aq. solution (50 ml) was added thereto under stirring. The insoluble matter was removed by filtration, the organic layer separated from the filtrate was analyzed by using HPLC. The result showed that the organic layer contained 0.38 g of the β-methyl derivative (β-methyl derivative: α-methyl derivative= 100:0). It was purified according to the method described in Example 16, affording the purified β-methyl derivative.

The melting point of the β-methyl derivative : 128∼129 °C

### Example 18

### Preparation of β-methyl derivative (methyl N-[(R)-2-[(3S, 4R)-3-[(R)-1-tert-butyldimethylsilyloxyethyl]-2-oxoazetidin-4-yl]propionyl]-N-phenylthiocarbamate)

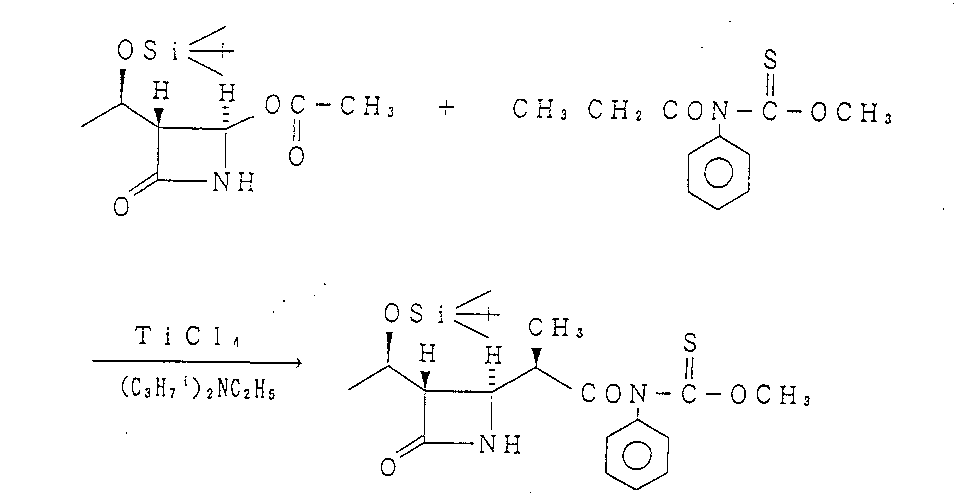

Methyl N-phenyl-N-propionylthiocarbamate (2.23 g, 10 mmol) solution in methylene chloride (30 ml) was cooled to 0°C, then titanium tetrachloride (1.9 g, 10 mmol) solution in methylene chloride (5 ml) was added to the solution. After aging for 15 min. at 0 °C, N,N-diisopropylethylamine (1.3 g, 10 mmol) solution in methylene chloride (5 ml) and (3R, 4R)-4-acetoxy-3-[(R)-1-tert-butyldimethylsilyloxyethyl]azetidin-2-one (1.44 g, 5 mmol) solution in methylene chloride (10 ml) were added to the mixture at the same temperature. After aging the reaction mixture for 1 hour at 0°C, it was warmed to 20 °C and further stirred for 3 hours. The reaction mixture was then cooled to 0°C and 10 % sodium hydrogen carbonate aq. solution (50 ml) was added thereto under stirring. The insoluble matter was removed by filtration, the organic layer separated from the filtrate was analyzed by using HPLC. The result showed that the organic layer contained 1.16 g of the β-methyl derivative (β-methyl derivative: α-methyl derivative= 72:28). It was purified according to the method described in Example 16, affording the purified β-methyl derivative.

The melting point of the β-methyl derivative : 100∼101 °C

### Example 19

### Preparation of α-methyl derivative (N-[(S)-2-[(3S, 4R)-3-[(R)-1-tert-butyldimethylsilyloxyethyl]-2-oxoazetidin-4-yl]propionyl]-N-(4-chlorophenyl)-p-toluenesulfonamide)

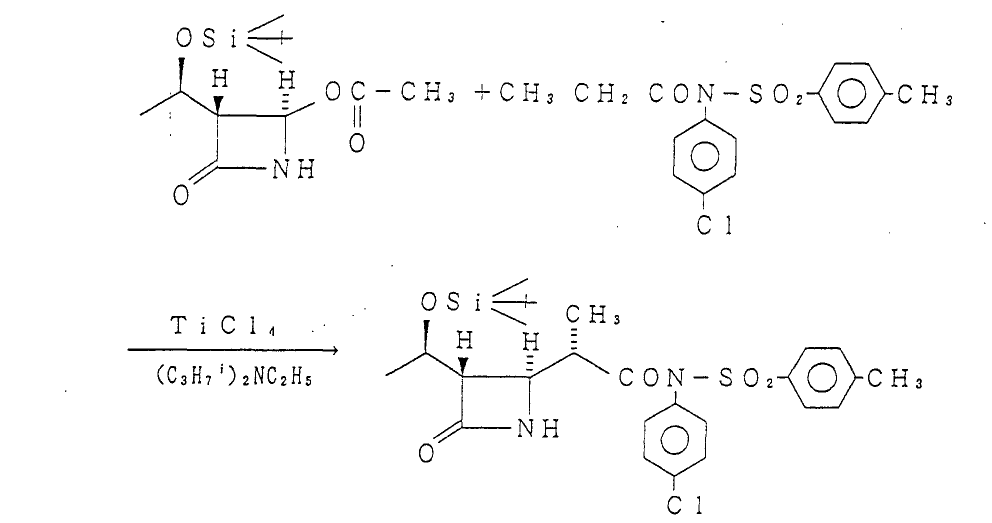

N-(4-chlorophenyl)-N-propionyl-p-toluenesulfonamide (676 mg, 2 mmol) solution in methylene chloride (2 ml) was cooled to 0 °C, then titanium tetrachloride/methylene chloride (1 M, 2 ml, 2 mmol) solution was added to the solution. After aging for 15 min. at 0°C, N,N-diisopropylethylamine (259 mg, 2 mmol) solution in methylene chloride (1 ml) and (3R, 4R)-4-acetoxy-3-[(R)-1-tert-butyldimethylsilyloxyethyl]azetidin-2-one (287.5 mg, 1 mmol) solution in methylene chloride (1 ml) were added to the solution at the same temperature. After aging the reaction mixture for 1 hour at 0°C, it was warmed to 20 °C and further stirred for 3 hours. The reaction mixture was then cooled to 0 °C and 10 % sodium hydrogen carbonate aq. solution (10 ml) was added thereto under stirring. The insoluble matter was removed by filtration, the organic layer separated from the filtrate was analyzed by using HPLC. The result showed that the organic layer contained 190 mg of the α-methyl derivative (α-methyl derivative: *β*-methyl derivative= 59:41). It was purified according to the method described in Example 16, affording the purified α-methyl derivative.

¹H NMR (270MHz, CDCl₃) δ of a α-methyl derivative: 0.03(6H,d), 0.84(9H,s), 1.01(3H,d), 1.14(3H,d), 2.2∼2.6(5H,m), 7.1∼7.9(8H,m), 3.65 ∼3.78(1H, m), 4.0 ∼4.15(1H, m), 5.90(1H, s)

### Example 20

### Preparation of α-methyl derivative (N-(S)-2-[(3S, 4R)-3-[(R)-1-tert butyldimethylsilyloxyethyl]-2-oxoazetidin-4-yl]propionyl]-N-isopropylbenzamide)

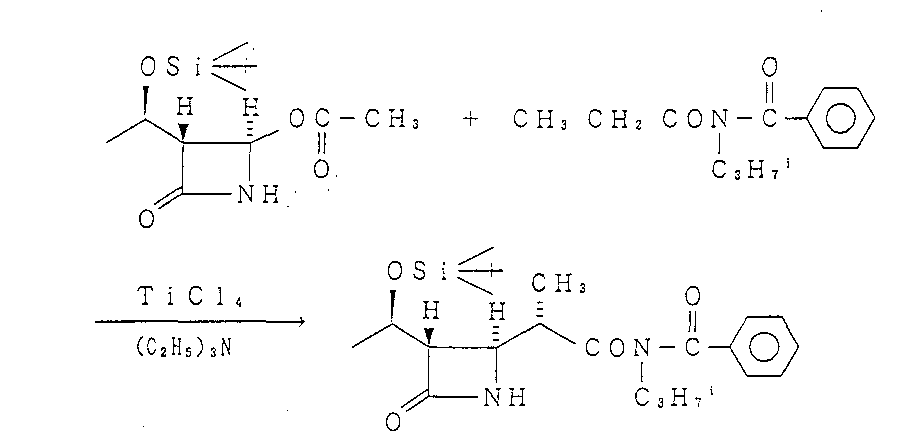

Titanium tetrachloride (0.43 g, 2.3 mmol) solution in methylene chloride (5 ml) was added to N-isopropyl-N-propionylbenzamide (0.5 g, 2.3 mmol) solution in methylene chloride (10 ml) at room temperature, then triethylamine (0.22 g, 2.1 mmol) and (3R, 4R)-4-acetoxy-3-[(R)-1-tert-butyldimethylsilyloxyethyl]azetidin-2-one (0.44 g, 1.52 mmol) were added to the mixture at the same temperature. The reaction mixture was stirred for 1 hour at 20 °C, then cooled to 0°C and added with 10 % sodium hydrogen carbonate aq. solution (50 ml) under stirring. The insoluble matter was removed by filtration, the organic layer separated from the filtrate was analyzed by using HPLC. The result showed that the organic layer contained 0.25 g of the α-methyl derivative (α-methyl derivative: *β*-methyl derivative= 95:5). It was purified according to the method described in Example 16, affording the purified α-methyl derivative.

1H NMR (270MHz, CDCl₃) *δ* of the α-methyl derivative: 0.06(6H,s), 0.87(9H,s), 0.99(3H,d), 1.06(3H,d), 1.38(6H, d), 2.6 ∼2.7(1H, m), 2.83(1H, dd), 3.76∼3.82(1H, m), 4.05∼4.18(1H, m), 4.45∼4. 66(1H, m), 6.16(1H,s), 7.3 ∼ 7.8(5H,m)

### Example 21

### Preparation of β-methyl derivative (N-[(R)-2[(3S, 4R)-3[(R)-1-tert-butyldimethylsilyloxyethyl]-2-oxoazetidin-4-yl]propionyl]-N-isopropyl-p toluenesulfonamide)

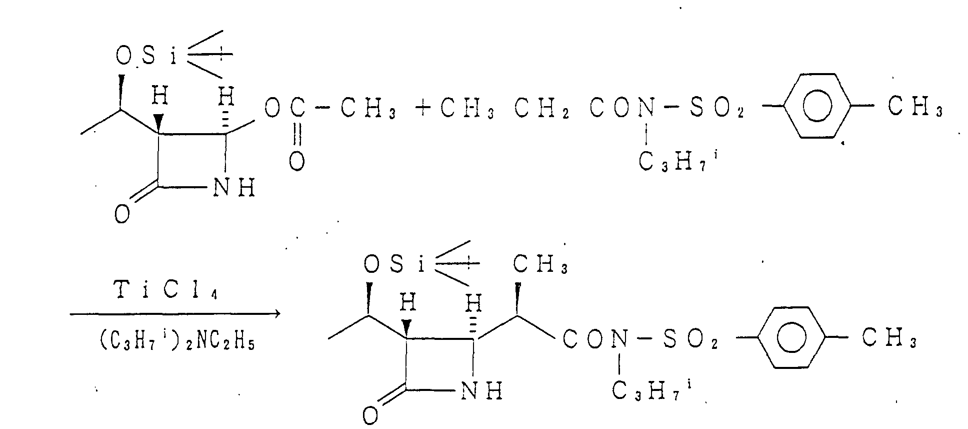

N-isopropyl-N-propionyl-p-toluenesulfonamide (2.69 g, 10 mmol) solution in methylene chloride (20 ml) was cooled to 0°C, then titanium tetrachloride (1.9 g, 10 mmol) solution in methylene chloride (2.5 ml) was added to the solution. After aging for 1 hour at 0°C, N,N-diisopropylethylamine (1.29 g, 10 mmol) solution in methylene chloride (2.5 ml) and (3R, 4R)-4-acetoxy-3-[(R)-1-tert-butyldimethylsilyloxyethyl]azetidin-2-one (1.44 g, 5 mmol) solution in methylene chloride (5 ml) were added to the mixture at the same temperature. After aging the reaction mixture for 2 hour at 0°C, it was warmed to 20 °C and further stirred for 3 hours. The reaction mixture was then cooled to 0°C and 10 % sodium hydrogen carbonate aq. solution (50 ml) was added thereto under stirring. The insoluble matter was removed by filtration and the filtrate was washed with water. The filtrate was dried with anhydrous magnesium sulfate, then concentrated under reduced pressure. The residue was purified by silica gel chromatography, affording 1.5 g of the mixture of the β-methyl derivative and the α-methyl derivative. From the analytical result of NMR (¹H), the ratio of the β-methyl derivative and the α-methyl derivative was 67 : 33. H NMR (270MHz, CDCl₃) δ of a β methyl derivative : 7.76(d,2H), 7.38(d,2H), 6.00(br S,1H), 4.55(m,1H), 4.15(m, 1H), 3.83(q, 1H), 3.59(m,1H), 2.89(br S, 1H), 1.43(q, 6H), 1.11(d,3H), 1.01(S,3H), 0.87(S,9H), 0.06(S,6H)

### Industrial Applicability

The process of the present invention is an industrially advantageous process using titanium compounds of the general formula [V] which are inexpensive and can be remored easily as titanium oxides.

Further, when R² is alkyl such as methyl group, it is possible to obtain the β-type derivatives selectivly, which are important as intermediates for the synthesis of carbapenem compounds, by adjusting the mole ratios or optionally selecting an auxiliary group.

## Claims

1. A process for preparing 4-substituted azetidinone derivatives of the general formula: 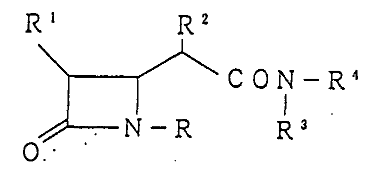 wherein R is hydrogen or an easily removable protecting group of nitrogen, R¹ is alkyl which can be substituted by hydroxy which can be protected or halogen, R² is hydrogen or alkyl, R³ is alkyl, trialkylsilyl, phenyl which can be substituted by alkyl, alkoxy, nitro or halogen, cycloalkyl, naphthyl, anthracenyl, fluorenyl, benzothiazolyl or naphthalimidyl and R⁴ is an electron withdrawing group or forming a ring together with R³, which comprises reacting an azetidinone derivative of the general formula: 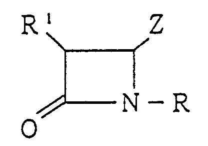 wherein R and R¹ are as defined above and Z is a leaving group, and an imide compound of the general formula: 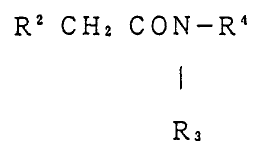 wherein R², R³ and R⁴ are as defined above, characterized in that the process is conducted in the presence of a titanium compound of the general formula:
Ti (Cl) ₙ (OR⁵)ₘ
wherein R⁵ is C₁ to C₃ alkyl, n and m are independently an integer from 0 to 4 provided n plus m always makes 4, and a base.

2. The process for the preparation according to claim 1, wherein R⁴ is a substituent represented by the formula: 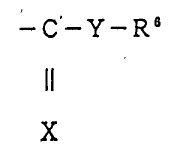 wherein X is oxygen, sulfur or NR⁷ (R⁷ is hydrogen, alkyl or phenyl); Y is oxygen, sulfur or NR⁸ (R⁸ is hydrogen, alkyl or phenyl); and R⁶ is alkyl, phenyl which can be substituted by alkyl, alkoxy, nitro or halogen, cycloalkyl, naphthyl, or forming 5- to 6-membered ring together with R³.

3. The process according to claim 1 or 2, wherein the ring formed with the following formula: 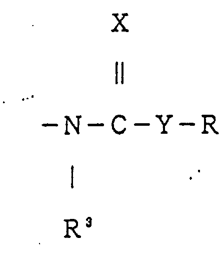 is a 5-membered ring represented by the formula: 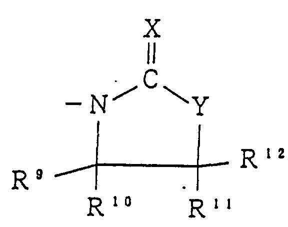 wherein R⁹, R¹⁰, R¹¹ and R¹² are independently hydrogen, alkyl, phenyl which can be substituted by lower alkyl, alkoxy, nitro or halogen, cycloalkyl, naphthyl, oxo or alkylene formed together with R⁹ and R¹⁰ or R¹¹ and R¹², or aromatic formed together with R⁹, R¹⁰, R¹¹ and R¹² and two adjacent carbon atoms connecting to these substituents; and, X and Y are as defined above.

4. The process according to claim 1, wherein R⁴ is a substituent of the general formula:
-SO₂ R¹³
wherein R¹³ is alkyl; haloalkyl; phenyl which can be substituted by alkyl, alkoxy, nitro or halogen; cycloalkyl or naphthyl or can form a ring together with R³.

5. The process according to claim 1 or 4, wherein the ring formed by the group of the general formula: 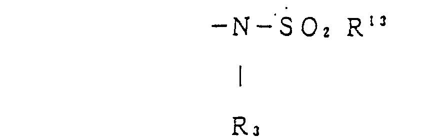 is a ring represented by the following formula: 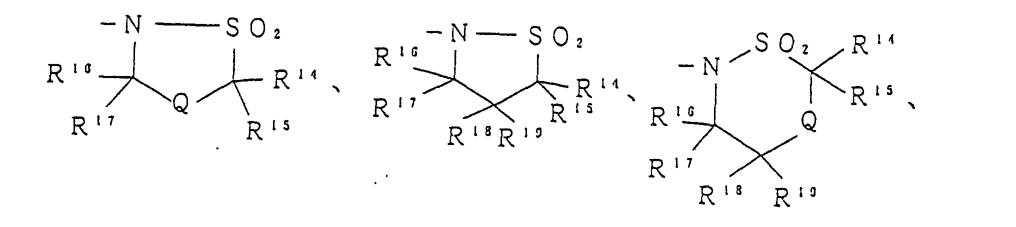 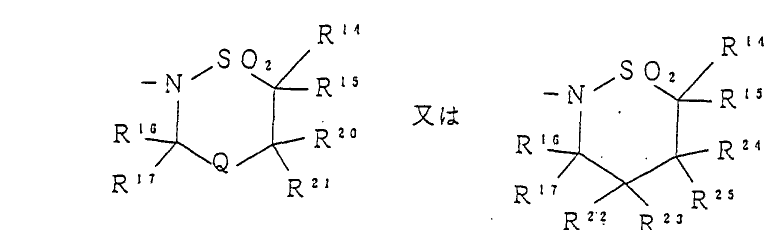 wherein R¹⁴, R¹⁵, R¹⁶, R¹⁷, R¹⁸, R¹⁹, R²⁰, R²¹, R²², R²³, R²⁴ and R²⁵ are independently hydrogen; alkyl; phenyl which can be substituted by alkyl, alkoxy, nitro or halogen; cycloalkyl; or R¹⁴ and R¹⁵, R¹⁶ and R¹⁷, R¹⁸ and R¹⁹, R²⁰ and R²¹, R²² and R²³ or R²⁴ and R²⁵ can respectively form oxo or alkylene group, and R¹⁴ and R²⁰, R¹⁶ and R²², R²² and R²⁴ or R¹⁴ and R²⁴ can form a ring, when R ¹⁷ and R¹⁹, R¹⁵ and R²¹, R¹⁷ and R²³, R²³ and R²⁵ or R¹⁵ and R²⁵ can respectively form double bond; Q is O, S or NR²⁶ (wherein R²⁶ is hydrogen, alkyl or phenyl).

6. The process according to claim 1, wherein R¹ is a substituent of the general formula: 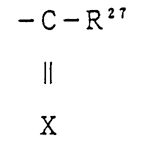 wherein X is as defined above. R²⁷ is alkyl; haloalkyl; phenyl which can be substituted by alkyl, alkoxy, nitro or halogen; cycloalkyl or naphthyl or can form a ring together with R³.

7. The process according to claim 1 or 6, wherein the ring formed from the group of the general formula: 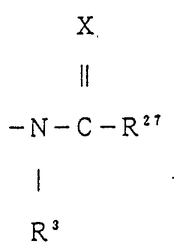 is a ring represented by the following formula: 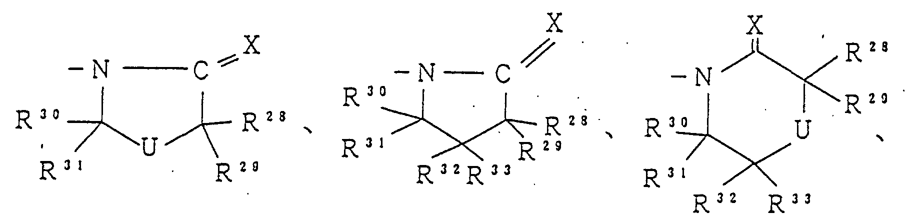 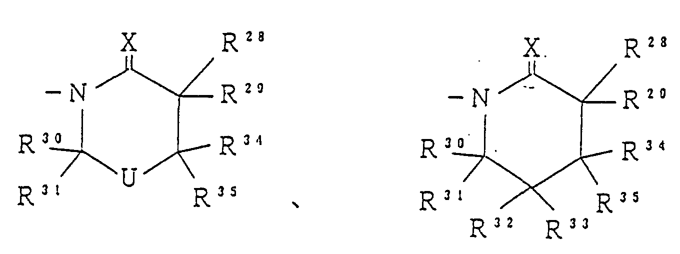 wherein X is as defined above; R²⁸, R²⁹, R³⁰, R³¹, R³², R³³, R³⁴ and R³⁵ are independently hydrogen; alkyl; pheyl which can be substituted with alkyl, alkoxy, nitro or halogen ; cycloalkyl; naphthyl; or R²⁸ and R²⁰, R³⁰ and R³¹, R ³² and R³³ or R³⁴ and R³⁵ can respectively form oxo or alkylene group, or R³⁰ and R³², R²⁸ and R³⁴ or R³² and R³⁴ can independently form a ring. When R³¹ and R³³, R²⁹ and R³⁵ or R³³ and R³⁵ can independently form a double bond; and U is O, S or NR³⁶ (wherein R³⁶ is hydrogen, alkyl or phenyl).

8. The process according to claim 1, wherein R⁴ is a substituent of the general formula:
SO₂―T― R³⁷
wherein T is 0, S or NR³⁸ (wherein R³⁸ is hydrogen or alkyl); R³⁷ is alkyl; phenyl which can be substituted by alkyl, alkoxy, nitro or halogen; cycloalkyl or naphthyl or can form 5- or 6-membered ring together with R³.

9. The process according to claim 1 or 8, wherein the ring of the general formula: 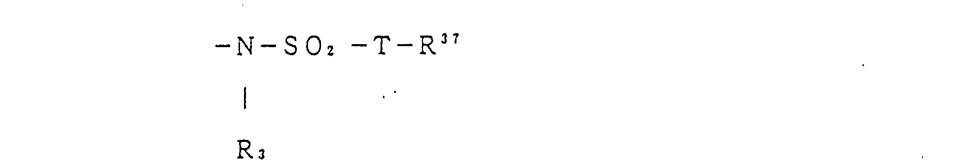 is a ring represented by the following formula: 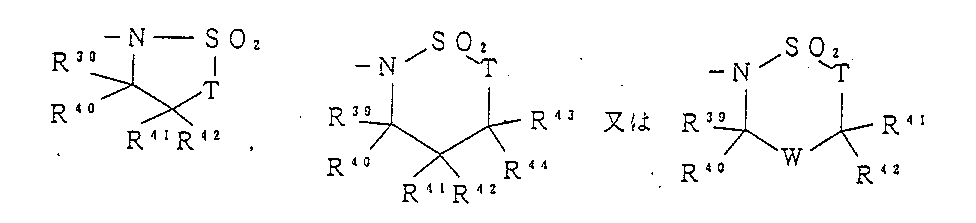 wherein R³⁹, R⁴⁰, R⁴¹, R⁴², R⁴³ and R⁴⁴ are independently hydrogen; alkyl; phenyl which can be substituted by alkyl, alkoxy, nitro or halogen; cycloalkyl; naphthyl; or R³⁹ and R⁴⁰, R⁴¹ and R⁴² or R⁴³ and R⁴⁴ can form oxo or alkylene, or R³⁹ and R⁴¹ or R⁴¹ and R⁴³ can form a ring respectively, when R⁴² and R⁴² or R⁴² and R⁴⁴ can form a double bond respectively; T is as defined above; W is O, S or NR⁴⁵ (wherein R⁴⁵ is hydrogen, alkyl or phenyl).

10. The process according to claim 1, wherein R⁴ is nitro.

## Patentansprüche

1. Ein Verfahren zur Herstellung 4-substituierter Azetidinon-derivate der allgemeinen Formel: 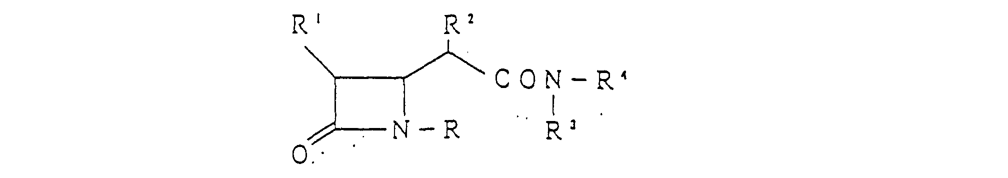 worin R Wasserstoff oder eine leicht zu entfernende Schutzgruppe von Stickstoff ist, R¹ Alkyl, welches mit Hydroxy substituiert sein kann, das geschützt sein kann, oder Halogen ist, R² Wasserstoff oder Alkyl ist, R³ Alkyl, Trialkylsilyl, Phenyl, welches mit Alkyl, Alkoxy, Nitro oder Halogen substituiert sein kann, Cycloalkyl, Naphthyl, Anthracenyl, Fluorenyl, Benzothiazolyl oder Naphthalimidyl ist, und R⁴ eine Elektronen-abziehen-de Gruppe ist oder zusammen mit R³ einen Ring bildet, welches ein Umsetzen eines Azetidinonderivats der allgemeinen Formel: 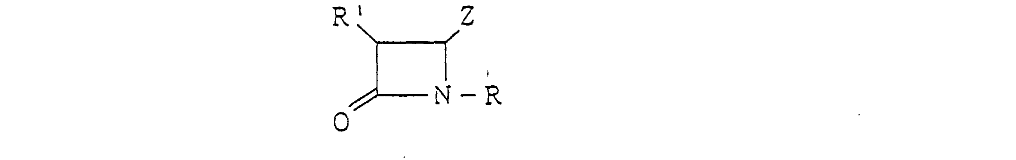 worin R und R¹ wie oben definiert sind und Z eine Abgangsgruppe ist, und einer Imidverbindung der allgemeinen Formel: 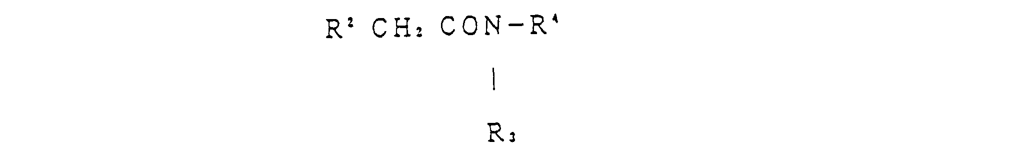 umfaßt, worin R², R³ und R⁴ so wie oben definiert sind, dadurch gekennzeichnet, daß das Verfahren in Gegenwart einer Titanverbindung der allgemeinen Formel:
Ti(Cl)ₙ(OR⁵)ₘ
worin R⁵ ein C₁-C₃-Alkyl ist, n und m unabhängig voneinander eine ganze Zahl von 0 bis 4 sind, unter der Voraussetzung, daß n plus m immer 4 ist, sowie einer Base durchgeführt wird.

2. Das Verfahren zur Herstellung gemäß Anspruch 1, worin R⁴ ein Substituent ist, welcher durch die Formel: 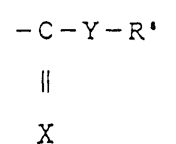 dargestellt wird, worin X Sauerstoff, Schwefel oder NR⁷ (R⁷ ist Wasserstoff, Alkyl oder Phenyl) ist; Y Sauerstoff, Schwefel oder NR⁸ (R⁸ ist Wasserstoff, Alkyl oder Phenyl) ist; und R⁶ Alkyl, Phenyl, welches mit Alkyl, Alkoxy, Nitro oder Halogen substituiert sein kann, Cycloalkyl, Naphthyl ist oder zusammen mit R³ einen 5- bis 6-gliedrigen Ring bildet.

3. Das Verfahren gemäß Anspruch 1 oder 2, worin der Ring, der mit der folgenden Formel 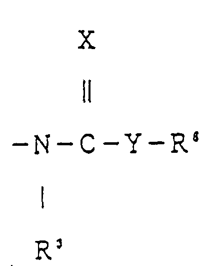 gebildet wird, ein 5-gliedriger Ring ist, der durch die Formel: 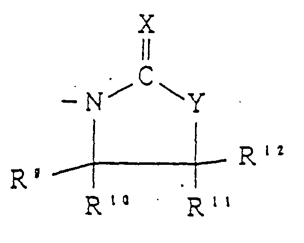 dargestellt wird, worin R⁹, R¹⁰, R¹¹ und R¹² unabhängig voneinander Wasserstoff, Alkyl, Phenyl, welches mit einem niederen Alkyl, Alkoxy, Nitro oder Halogen substituiert sein kann, Cycloalkyl, Naphthyl, Oxo oder Alkylen, die zusammen mit R⁹ und R¹⁰ oder R¹¹ und R¹² gebildet werden, oder aromatisch sind und zusammen mit R⁹, R¹⁰, R¹¹ und R¹² sowie zwei benachbarten Kohlenstoffatomen gebildet werden, welche diese Substituenten verbinden; und X und Y so wie oben definiert sind.

4. Das Verfahren gemäß Anspruch 1, worin R⁴ ein Substituent der allgemeinen Formel:
-SO₂R¹³
ist, worin R¹³ Alkyl, Halogenalkyl, Phenyl, das mit Alkyl, Alkoxy, Nitro oder Halogen substituiert sein kann, Cycloalkyl oder Naphthyl ist oder zusammen mit R³ einen Ring bilden kann.

5. Das Verfahren gemäß Anspruch 1 oder 4, worin der Ring, der durch die Gruppe der allgemeinen Formel: 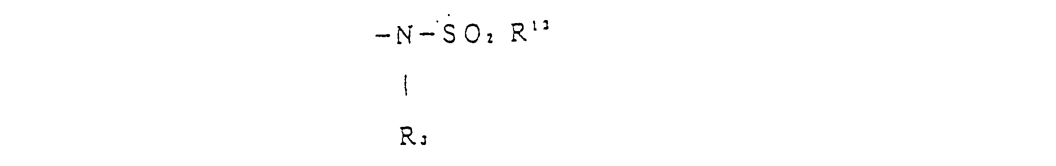 gebildet wird, ein Ring ist, der durch die folgende Formel dargestellt wird: 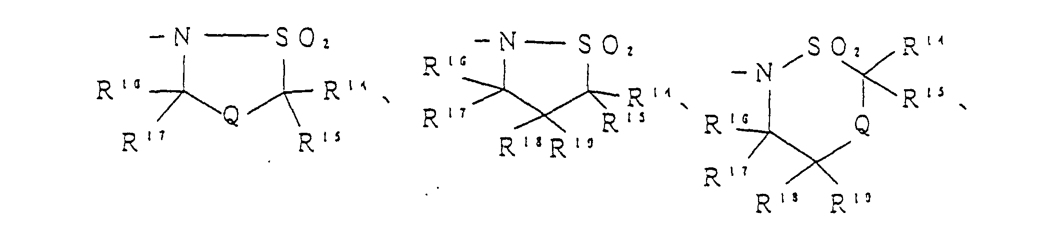 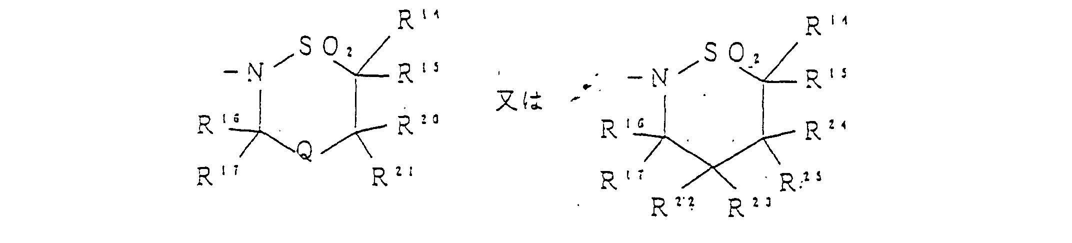 worin R¹⁴, R¹⁵, R¹⁶, R¹⁷, R¹⁸, R¹⁹, R²⁰, R²¹, R²², R²³, R²⁴ und R²⁵ unabhängig voneinander Wasserstoff, Alkyl, Phenyl, welches mit Alkyl, Alkoxy, Nitro oder Halogen substituiert sein kann, Cycloalkyl sind, oder R¹⁴ und R¹⁵, R¹⁶ und R¹⁷, R¹⁸ und R¹⁹, R²⁰ und R²¹, R²² und R²³ oder R²⁴ und R²⁵ entsprechend eine Oxo- oder Alkylengruppe bilden können, und R¹⁴ und R²⁰, R¹⁶ und R²², R²² und R²⁴ oder R¹⁴ und R²⁴ einen Ring bilden können, wenn R¹⁷ und R¹⁹, R¹⁵ und R²¹, R¹⁷ und R²³, R²³ und R²⁵ oder R¹⁵ und R²³ entsprechend eine Doppelbindung bilden können; Q O, S oder NR²⁶ ist (worin R²⁶ Wasserstoff, Alkyl oder Phenyl ist).

6. Das Verfahren gemäß Anspruch 1, worin R⁴ ein Substituent der allgemeinen Formel: 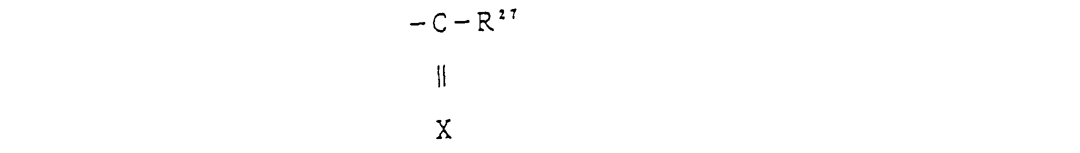 ist, worin X wie oben definiert ist, R²⁷ Alkyl, Halogenalkyl, Phenyl, welches mit Alkyl, Alkoxy, Nitro oder Halogen substituiert sein kann, Cycloalkyl oder Naphthyl ist oder zusammen mit R³ einen Ring bilden kann.

7. Das Verfahren gemäß Anspruch 1 oder 6, worin der Ring, der aus der Gruppe der allgemeinen Formel: 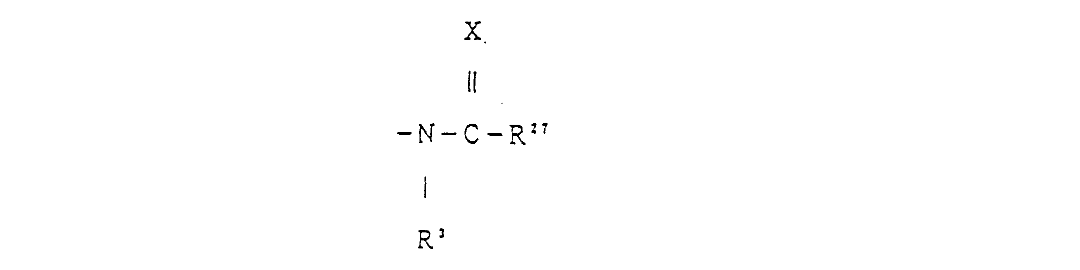 gebildet wird, ein Ring ist, der durch die folgende Formel dargestellt wird: 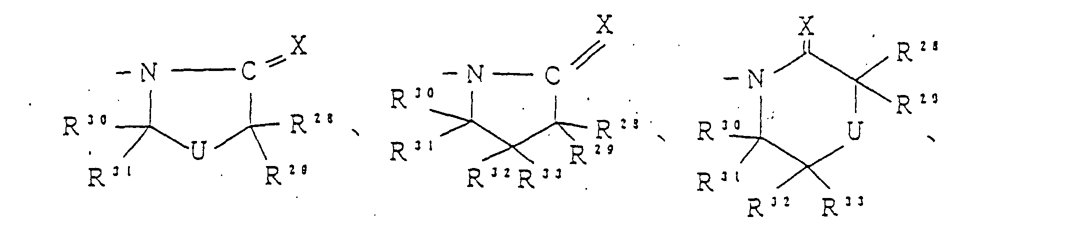 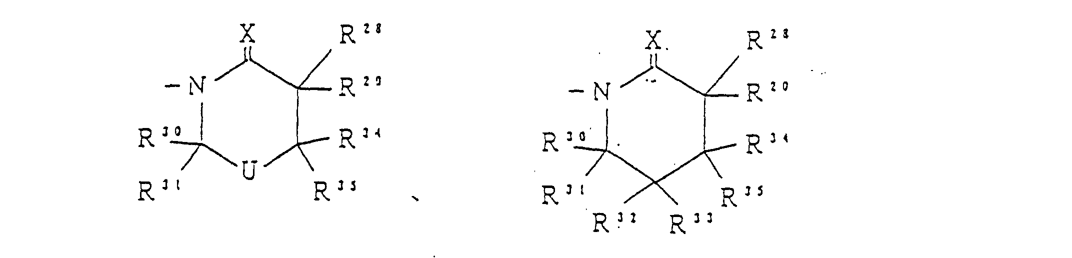 worin X wie oben definiert ist, R²⁸, R²⁹, R³⁰, R³¹, R³², R³³, R³⁴ und R³⁵ unabhängig voneinander Wasserstoff, Alkyl, Phenyl, welches mit Alkyl, Alkoxy, Nitro oder Halogen substituiert sein kann, Cycloalkyl, Naphthyl sind, oder R²⁸ und R²⁰, R³⁰ und R³¹, R³² und R³³ oder R³⁴ und R³⁵ entsprechend eine Oxo- oder Alkylengruppe bilden können, oder R³⁰ und R³², R²⁸ und R³⁴ oder R³² und R³⁴ unabhängig voneinander einen Ring bilden können, wenn R³¹ und R³³, R²⁹ und R³⁵ oder R³³ und R³⁵ unabhängig voneinander eine Doppelbindung bilden können, und U O, S oder NR³⁶ ist (worin R³⁶ Wasserstoff, Alkyl oder Phenyl ist).

8. Das Verfahren gemäß Anspruch 1, worin R⁴ ein Substituent der allgemeinen Formel:
SO₂ - T - R³⁷
ist, worin T O, S oder NR³⁸ ist (worin R³⁸ Wasserstoff oder Alkyl ist), R³⁷ Alkyl, Phenyl, welches mit Alkyl, Alkoxy, Nitro oder Halogen substituiert sein kann, Cycloalkyl oder Naphthyl ist oder zusammen mit R³ einen 5- oder 6-gliedrigen Ring bilden kann.

9. Das Verfahren gemäß Anspruch 1 oder 8, worin der Ring mit der allgemeinen Formel: 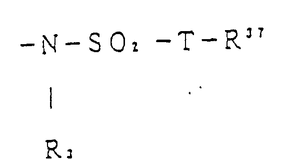 ein Ring ist, der durch die folgende Formel dargestellt wird: 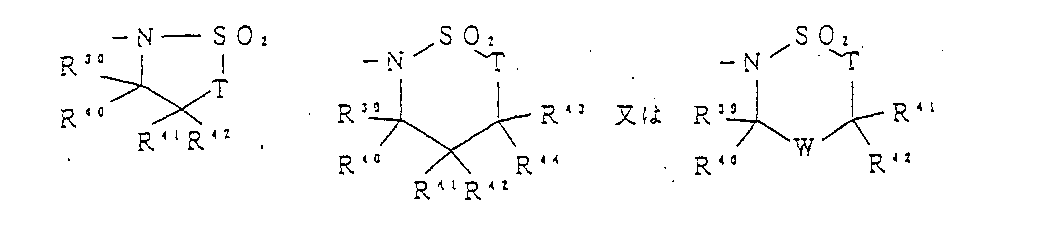 worin R³⁹, R⁴⁰, R⁴¹, R⁴², R⁴³ und R⁴⁴ unabhängig voneinander Wasserstoff, Alkyl, Phenyl, welches mit Alkyl, Alkoxy, Nitro oder Halogen substituiert sein kann, Cycloalkyl, Naphthyl sind, oder R³⁹ und R⁴⁰, R⁴¹ und R⁴² oder R⁴³ und R⁴⁴ ein Oxo oder Alkylen bilden können, oder R³⁹ und R⁴¹ oder R⁴¹ und R⁴³ entsprechend einen Ring bilden können, wenn R⁴² und R⁴² oder R⁴² und R⁴⁴ entsprechend eine Doppelbindung bilden können, T wie oben definiert ist, W O, S oder NR⁴⁵ ist (worin R⁴⁵ Wasserstoff, Alkyl oder Phenyl ist).

10. Das Verfahren gemäß Anspruch 1, worin R⁴ Nitro ist.

## Revendications

1. Procédé de préparation de dérivés d'azétidinone substitués en position 4 de formule générale : 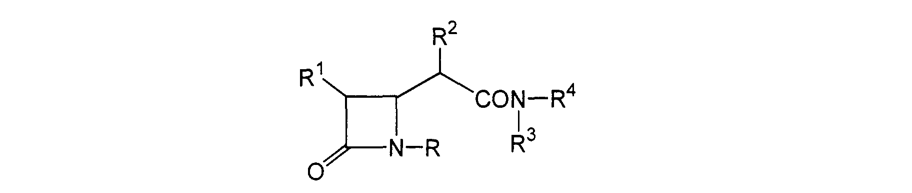 dans laquelle R est l'hydrogène ou un groupe protecteur de l'azote pouvant être éliminé facilement, R¹ est un groupe alkyle qui peut être substitué par un groupe hydroxy qui peut être protégé ou par un halogène, R² est l'hydrogène ou un groupe alkyle, R³ est un groupe alkyle, trialkylsilyle, phényle qui peut être substitué par un groupe alkyle, alcoxy, nitro ou halogéno, cycloalkyle, naphtyle, anthracényle, fluorényle, benzothiazolyle, naphtalimidyle et R⁴ est un groupe attracteur d'électrons ou formant un cycle avec R³, qui comprend la réaction d'un dérivé d'azétidinone de formule générale : 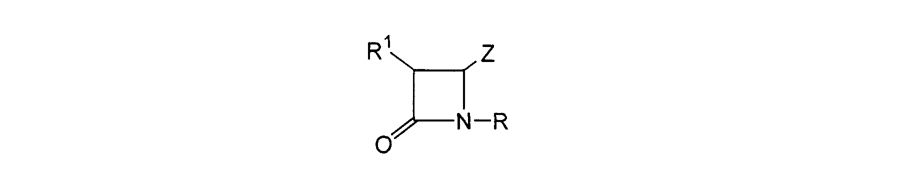 dans laquelle R et R¹ sont tels que définis ci-dessus, et Z est un groupe partant, et d'un composé imide de formule générale : 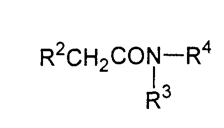 dans laquelle R², R³ et R⁴ sont tels que définis ci-dessus, caractérisé en ce que le procédé est effectué en présence d'un composé de titane de formule générale :
Ti(Cl)ₙ(OR⁵)ₘ
dans laquelle R⁵ est un groupe alkyle en C₁ à C₃; n et m sont indépendamment un entier de 0 à 4 sous réserve que n plus m valent toujours 4, et d'une base.

2. Procédé de préparation selon la revendication 1, caractérisé en ce que R⁴ est un substituant représenté par la formule : 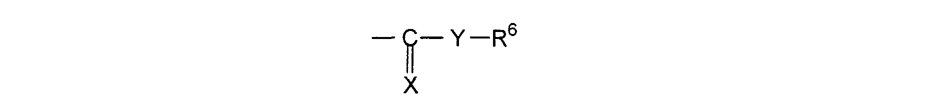 dans laquelle X est l'oxygène, le soufre ou NR⁷ (R⁷ est l'hydrogène, un groupe alkyle ou phényle); Y est l'oxygène, le soufre ou NR⁸ (R⁸ est l'hydrogène, un groupe alkyle ou phényle) ; et R⁶ est un groupe alkyle, phényle qui peut être substitué par un groupe alkyle, alcoxy, nitro ou halogéno, cycloalkyle, naphtyle, ou formant un cycle à 5 ou 6 chaînons avec R³.

3. Procédé selon la revendication 1 ou 2, caractérisé en ce que le cycle formé avec la formule suivante : 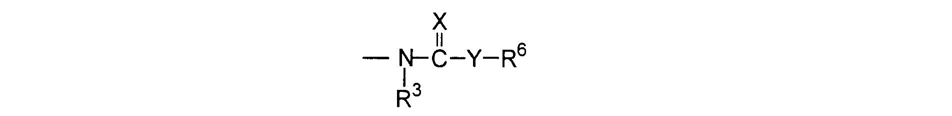 est un cycle à 5 chaînons représenté par la formule : 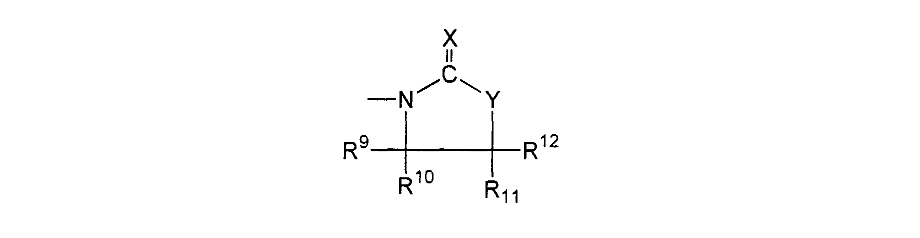 dans laquelle R⁹, R¹⁰, R¹¹ et R¹² sont indépendamment l'hydrogène, un groupe alkyle, phényle qui peut être substitué par un groupe alkyle inférieur, alcoxy, nitro ou halogéno, cycloalkyle, naphtyle, oxo ou alkylène formé avec R⁹ et R¹⁰ ou R¹¹ et R¹², ou aromatique formé avec R⁹, R¹⁰, R¹¹ et R¹² et deux atomes de carbone adjacents reliés à ces substituants ; et X et Y sont tels que définis ci-dessus.

4. Procédé selon la revendication 1, caractérisé en ce que R⁴ est un substituant de formule générale :
-SO₂R¹³
dans laquelle R¹³ est un groupe alkyle ; haloalkyle ; phényle qui peut être substitué par un groupe alkyle, alcoxy, nitro ou halogéno ; cycloalkyle ou naphtyle ou peut former un cycle avec R³.

5. Procédé selon la revendication 1 ou 4, caractérisé en ce que le cycle formé par le groupe de formule générale : 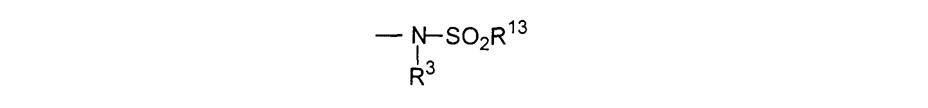 est un cycle représenté par les formules suivantes : 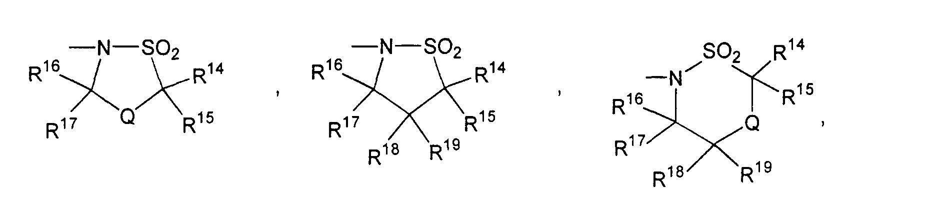 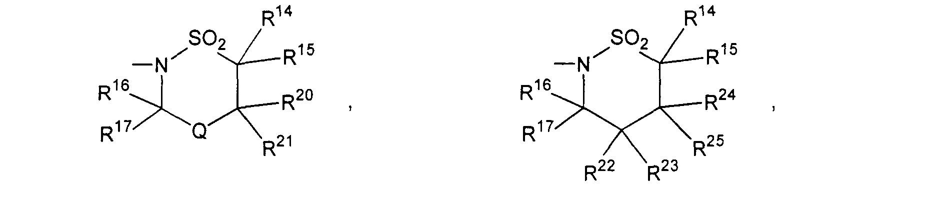 dans lesquelles R¹⁴, R¹⁵, R¹⁶, R¹⁷, R¹⁸, R¹⁹, R²⁰, R²¹, R²², R²³, R²⁴ et R²⁵ sont indépendamment l'hydrogène ; un groupe alkyle ; phényle qui peut être substitué par un groupe alkyle, alcoxy, nitro ou halogéno; cycloalkyle ; ou R¹⁴ et R¹⁵, R¹⁶ et R¹⁷, R¹⁸ et R¹⁹, R²⁰ et R²¹, R²² et R²³ ou R²⁴ et R²⁵ peuvent former respectivement un groupe oxo ou alkylène, et R¹⁴ et R²⁰, R¹⁶ et R²², R²² et R²⁴ ou R¹⁴ et R²⁴ peuvent former un cycle, quand R¹⁷ et R¹⁹, R¹⁵ et R²¹, R¹⁷ et R²³, R²³ et R²⁵ ou R¹⁵ et R²⁵ peuvent former respectivement une double liaison ; Q est O, S ou NR²⁶ (où R²⁶ est l'hydrogène, un groupe alkyle ou phényle).

6. Procédé selon la revendication 1, caractérisé en ce que R⁴ est un substituant de formule générale : 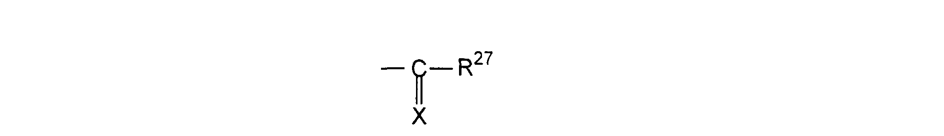 dans laquelle X est tel que défini ci-dessus, R²⁷ est un groupe alkyle; haloalkyle ; phényle qui peut être substitué par un groupe alkyle, alcoxy, nitro ou halogéno ; cycloalkyle ou naphtyle, ou peut former un cycle avec R³.

7. Procédé selon la revendication 1 ou 6, caractérisé en ce que le cycle formé par le groupe de formule générale : 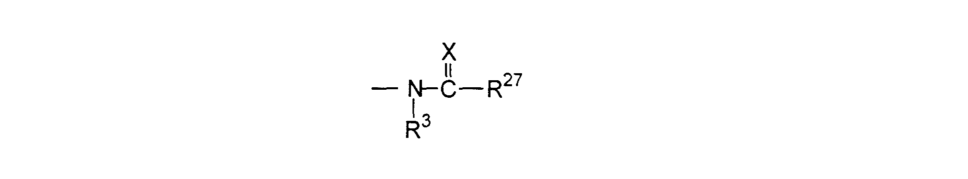 est un cycle représenté par les formules suivantes : 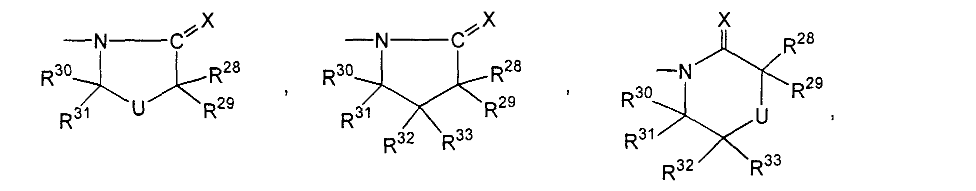 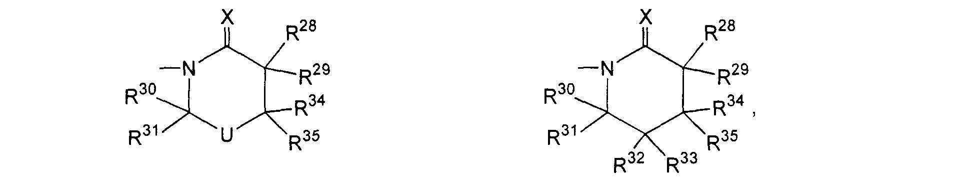 dans lesquelles X est tel que défini ci-dessus ; R²⁸, R²⁹, R³⁰, R³¹, R³², R³³, R³⁴ et R³⁵ sont indépendamment l'hydrogène ; un groupe alkyle ; phényle qui peut être substitué par un groupe alkyle, alcoxy, nitro ou halogéno ; cycloalkyle ; naphtyle ; ou R²⁸ et R²⁹, R³⁰ et R³¹, R³² et R³³ ou R³⁴ et R³⁵ peuvent former respectivement un groupe oxo ou alkylène, ou R³⁰ et R³², R²⁸ et R³⁴ ou R³² et R³⁴ peuvent former indépendamment un cycle, quand R³¹ et R³³, R²⁹ et R³⁵ ou R³³ et R³⁵ peuvent former indépendamment une double liaison ; et U est O, S ou NR³⁶ (où R³⁶ est l'hydrogène, un groupe alkyle ou phényle).

8. Procédé selon la revendication 1, caractérisé en ce que R⁴ est un substituant de formule générale :
SO₂-T-R³⁷
dans laquelle T est O, S ou NR³⁸ (où R³⁸ est l'hydrogène ou un groupe alkyle) ; R³⁷ est un groupe alkyle ; phényle qui peut être substitué par un groupe alkyle, alcoxy, nitro ou halogéno ; cycloalkyle ou naphtyle ou peut former un cycle à 5 ou 6 chaînons avec R³.

9. Procédé selon la revendication 1 ou 8, caractérisé en ce que le cycle de la formule générale : 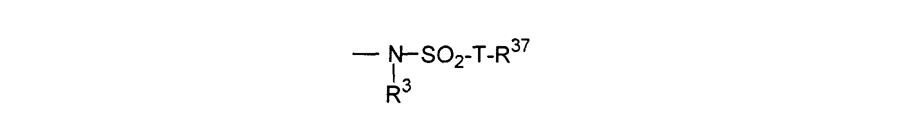 est un cycle représenté par les formules suivantes : 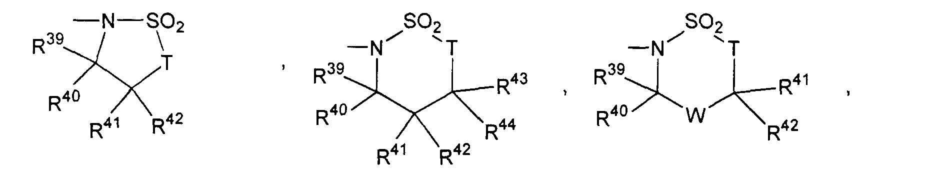 dans lesquelles R³⁹, R⁴⁰, R⁴¹, R⁴², R⁴³ et R⁴⁴ sont indépendamment l'hydrogène ; un groupe alkyle ; phényle qui peut être substitué par un groupe alkyle, alcoxy, nitro ou halogéno ; cycloalkyle ; naphtyle ; ou R³⁹ et R⁴⁰, R⁴¹ et R⁴² ou R⁴³ et R⁴⁴ peuvent former un groupe oxo ou alkylène, ou R³⁹ et R⁴¹ ou R⁴¹ et R⁴³ peuvent former respectivement un cycle, quand R⁴⁰ et R⁴² ou R⁴² et R⁴⁴ peuvent former respectivement une double liaison ; T est tel que défini ci-dessus ; W est O, S ou NR⁴⁵ (où R⁴⁵ est l'hydrogène, un groupe alkyle ou phényle).

10. procédé selon la revendication 1, caractérisé en ce que R⁴ est un groupe nitro.
